(19) **Europäisches Patentamt** / **European Patent Office** / **Office européen des brevets**

(11) **EP 4 558 785 B1**

(12) **FASCICULE DE BREVET EUROPEEN**

(45) Date de publication et mention
de la délivrance du brevet:
**25.03.2026 Bulletin 2026/13**

(21) Numéro de dépôt: **23741001.4**

(22) Date de dépôt: **07.07.2023**

(51) Classification Internationale des Brevets (IPC):
**G01B 9/02055** (2022.01)   **A61B 5/00** (2006.01)
**G01B 9/02** (2022.01)   **G01B 9/02091** (2022.01)
**G01N 21/47** (2006.01)   **G02B 21/36** (2006.01)

(52) Classification Coopérative des Brevets (CPC):
**G01B 9/02075; A61B 5/0062; A61B 5/0077;
G01B 9/02083; G01B 9/02091; G01N 21/4795;
G02B 21/365;** A61B 5/0035

(86) Numéro de dépôt international:
**PCT/EP2023/068840**

(87) Numéro de publication internationale:
**WO 2024/017670 (25.01.2024 Gazette 2024/04)**

(54) **PROCÉDÉS ET SYSTÈMES DE CARACTÉRISATION OPTIQUE D'UN MILIEU VOLUMIQUE ET DIFFUSANT**

VERFAHREN UND SYSTEME ZUR OPTISCHEN CHARAKTERISIERUNG EINES STREUENDEN VOLUMENMEDIUMS

METHODS AND SYSTEMS FOR OPTICAL CHARACTERISATION OF A BULK SCATTERING MEDIUM

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorité: **18.07.2022 FR 2207334**

(43) Date de publication de la demande:
**28.05.2025 Bulletin 2025/22**

(73) Titulaires:
• **Centre National de la Recherche Scientifique**
**75016 Paris (FR)**
• **Ecole Supérieure de Physique et de Chimie
Industrielles de la Ville de Paris**
**75005 Paris 5 (FR)**

(72) Inventeurs:
• **AUBRY, Alexandre**
**94200 Ivry-sur-Seine (FR)**
• **BAROLLE, Victor**
**75010 Paris (FR)**
• **BALONDRADE, Paul**
**75014 Paris (FR)**
• **FINK, Mathias Alexandre**
**92190 Meudon (FR)**
• **BOCCARA, Albert Claude**
**75006 Paris (FR)**
• **NAJAR, Ulysse**
**94110 Arcueil (FR)**

(74) Mandataire: **IPAZ**
**Bâtiment Platon
Parc Les Algorithmes
91190 Saint-Aubin (FR)**

(56) Documents cités:
**US-A1- 2021 310 787**

## Description

### Domaine technique de l'invention

La présente description concerne des procédés et systèmes de caractérisation optique d'un milieu volumique et diffusant. La présente description s'applique notamment, mais non exclusivement, à l'imagerie biomédicale pour la caractérisation de tissus biologiques.

### Etat de la technique

La caractérisation optique de milieux volumiques et diffusants tels que les tissus biologiques est une discipline de recherche à la croisée de la physique et de la biologie. L'objectif est d'imager les structures qui composent les tissus biologiques avec une résolution micrométrique limitée par la diffraction i.e. de l'ordre de la longueur d'onde optique. Le principe général est le suivant : une onde lumineuse illumine l'échantillon, puis la lumière diffusée par les structures du milieu est collectée par un ensemble de lentilles afin de former une image agrandie du tissu, sur une caméra par exemple. Les microscopes connus du grand public sont généralement des microscopes en transmission, du fait de leur simplicité de conception et d'utilisation. Cependant, ils sont limitants pour l'imagerie in-vivo puisqu'il est nécessaire de placer la source et la caméra de part et d'autre de l'échantillon. L'approche en réflexion est ainsi privilégiée pour des applications qui comprennent notamment une imagerie in-vivo microscopique.

Pour des échantillons peu épais (L < 50 $\mu$m), l'onde rétrodiffusée par l'échantillon est fidèle à la réflectivité de l'objet ; seule l'ouverture numérique du système optique détermine la résolution axiale et transverse de l'image microscopique. Cependant, dès lors que l'on souhaite imager des tissus en profondeur, le phénomène de diffusion multiple devient limitant. En effet, les échantillons biologiques sont des milieux hétérogènes dont l'indice optique fluctue sur différentes échelles spatiales. Ces inhomogénéités d'indice non seulement dégradent la propagation de l'onde lumineuse (phénomène d'aberrations) ce qui a pour effet de diminuer le contraste et la résolution des images, mais limitent également le nombre de photons utiles pouvant être collectés (phénomène de diffusion multiple). La contribution de diffusion simple diminuant de manière exponentielle avec la profondeur, il devient impossible de discriminer celle-ci des contributions des photons aberrés et multiplement diffusés par l'échantillon au-delà d'une distance caractéristique correspondant au libre parcours moyen de diffusion $l_s$ (typiquement 50 - 100 $\mu$m dans les tissus).

Afin de pallier cette problématique, deux types de microscope ont vu le jour : le microscope confocal au début des années 1960 ainsi que le microscope interférométrique au début des années 1990. Le microscope confocal permet, par un filtrage spatial des photons diffusés, d'augmenter la résolution ainsi que la profondeur de pénétration du microscope classique (typiquement 100- 300 $\mu$m). Le microscope interférométrique et particulièrement l'OCT (acronyme anglais d' « *Optical Coherence Tomography* ») ajoute un filtrage temporel au filtrage spatial du microscope confocal. Cela permet d'augmenter encore les performances accessibles jusqu'à quelques $l_s$ (soit ~ 100 - 500 $\mu$m). Cependant, malgré le fenêtrage spatio-temporel des photons, les performances du microscope interférométrique en termes de résolution et de profondeur de pénétration sont toujours limitées par les phénomènes d'aberrations et de diffusion multiple.

Une problématique générale reste ainsi d'imager des échantillons biologiques en trois dimensions, au-delà de quelques $l_s$ avec une résolution seulement limitée par la diffraction. Au début des années 2000, inspirés de travaux pionniers en astronomie, des méthodes de correction d'aberrations se sont développées pour la microscopie optique. En astronomie, les fluctuations d'indice des couches atmosphériques créent des distorsions de front d'onde et dégradent la qualité des images du ciel formées depuis la Terre. Des méthodes dites d'optique adaptative, basées sur la mesure du front d'onde associée à un dispositif de contrôle de celui-ci (typiquement un réseau de miroirs déformables) permettent de compenser la distorsion du front d'onde et donc améliorer la résolution. Pour cela, une étoile guide ou bien un point brillant projeté dans le ciel est utilisé afin d'optimiser la correction. Ces techniques d'optique adaptative ont ensuite été transposées à la microscopie optique avec l'avènement des miroirs déformables de petites tailles ainsi que des SLM (acronyme anglais pour « *Spatial Light Modulator* »). Ces méthodes comportent cependant un certain nombre d'inconvénients. Tout d'abord, le temps de décorrélation des tissus biologiques impose une compensation rapide des aberrations et limite le nombre de degrés de liberté angulaires et/ou spatiaux des dispositifs de mesure et contrôle du front d'onde. L'optique adaptative dans les milieux biologiques se restreint donc à une compensation des ordres d'aberration relativement faibles. Par ailleurs, un facteur encore plus limitant est le champ de vision restreint sur lequel la compensation des aberrations est valable. Il correspond à ce qui est communément appelé une zone isoplanétique, i.e. une zone jusqu'à laquelle et depuis laquelle les fronts d'onde incident et réfléchi subissent les mêmes distorsions. Or la taille de ces zones isoplanétiques devient inférieure à une dizaine de micromètre à une profondeur de l'ordre d'un libre parcours moyen de transport $l_t$ (typiquement 1 mm dans les tissus biologiques). L'accès à une correction pour l'ensemble du champ de vision nécessite de répéter le processus d'optique adaptative pour chaque zone isoplanétique, ce qui rend illusoire une imagerie hautement résolue sur de grands champs de vision et à grande profondeur. Pour contrer la taille limitée des zones d'isoplanétisme, des dispositifs d'optique adaptative dits multi-conjugués ont été développés mais ils sont particulièrement complexes dans leurs réalisations expérimentales. Enfin, la diffusion multiple profonde (i.e. au-delà d'un libre parcours moyen de transport $l_t$) reste un problème qui n'est pas adressé par l'optique adaptative.

Une troisième voie qui ne repose ni sur la génération d'une étoile artificielle, ni sur une optimisation du front d'onde basée sur un critère de la qualité de l'image a été développée. Il s'agit d'une approche matricielle de l'imagerie optique et de la correction des aberrations. L'approche matricielle de la propagation des ondes lumineuses au sein des milieux hétérogènes a été développée tout d'abord en transmission, à des fins notamment de communication à travers des milieux fortement diffusants - voir article de S. M. Popoff *et al.* [Réf. 1].

Récemment, l'approche matricielle a été développée en réflexion pour l'imagerie à travers les milieux fortement diffusants - voir article de A. Badon *et al.* [Réf. 2]. Cette approche comprend la détermination expérimentale d'une matrice de réflexion « *plan focal* » ou « *focalisée* » fenêtrée temporellement et définie entre un plan source et un plan image, conjugués avec un plan focal objet d'un objectif de microscope.

En pratique, dans le montage expérimental principal décrit dans [réf. 2], un faisceau laser issu d'une source laser femtoseconde est mis en forme spatialement par un modulateur spatial de lumière (SLM) agissant comme un réseau de diffraction dynamique. Un ensemble d'ondes planes est alors émis par le SLM, les ondes planes étant focalisées en différents points de focalisation repérés chacun par un vecteur $\mathbf{r_{in}}$ du plan focal objet de l'objectif de microscope. Pour chaque point de focalisation $\mathbf{r_{in}}$, le champ réfléchi $E_r(\mathbf{u_{out}}, \mathbf{r_{in}}, t)$ est collecté au travers du même objectif de microscope et interfère avec une onde de référence $E_0(\mathbf{u_{out}}, \mathbf{0}, t)$ sur un dispositif d'acquisition bidimensionnelle, par exemple une caméra CCD, agencé dans un plan conjugué avec le plan pupille de l'objectif de microscope et repéré par le vecteur $\mathbf{u_{out}}$. La figure d'interférence entre ces deux ondes, intégrée sur le temps $t$ donne accès aux coefficients $R(\mathbf{u_{out}}, \mathbf{r_{in}})$ d'une colonne de la matrice de réflexion $\mathbf{R_{ur}}$ fenêtrée temporellement :

[Math 1]

$$R(\mathbf{u_{out}}, \mathbf{r_{in}}) = \int dt \ E_r(\mathbf{u_{out}}, \mathbf{r_{in}}, \mathrm{t}) \times E_0^*(\mathbf{u_{out}}, \mathbf{0}, \mathrm{t} + \tau)$$

où le symbole * désigne la matrice conjuguée. En pratique, l'amplitude et la phase de chaque coefficient $R(\mathbf{u_{out}}, \mathbf{r_{in}})$ sont enregistrées par interférométrie à décalage de phase. Le temps de vol $\tau$ est contrôlé par la longueur du bras de référence de l'interféromètre au moyen d'un miroir dont la position est ajustée par un actuateur piézoélectrique (PZT). Le temps de vol est ajusté au temps balistique pour la plupart des applications envisagées, de telle sorte à éliminer une majeure partie des photons multiplement diffusés et ne conserver que les photons simplement diffusés par les réflecteurs contenus dans le plan focal de l'objectif de microscope. Pour chaque point $\mathbf{r_{in}}$ de focalisation en entrée dans le plan focal, le coefficient de réflexion $R(\mathbf{u_{out}}, \mathbf{r_{in}})$ est enregistré dans un plan conjugué avec celui de la pupille de l'objectif de microscope en sortie (repéré par le vecteur $\mathbf{u_{out}}$). Une transformée de Fourier bidimensionnelle sur la coordonnée $\mathbf{u_{out}}$ permet de déterminer le coefficient de réflexion $R(\mathbf{r_{out}}, \mathbf{r_{in}})$ dans un plan conjugué avec la plan focal en sortie repéré par le vecteur $\mathbf{r_{out}}$. Pour chaque point $\mathbf{r_{in}}$ de focalisation en entrée dans le plan focal, le coefficient de réflexion $R(\mathbf{r_{out}}, \mathbf{r_{in}})$ est enregistré et stocké le long d'un vecteur colonne. Finalement, l'ensemble des vecteurs colonnes forme la matrice de réflexion $\mathbf{R_{rr}}$ dans le plan focal. On obtient ainsi une matrice de réflexion focalisée fenêtrée temporellement dont les éléments diagonaux ($\mathbf{r_{in}} = \mathbf{r_{out}}$) forment une coupe « *en face* » de l'image de l'échantillon telle qu'elle serait obtenue en OCT plein champ (pour une description de l'imagerie OCT plein champ, voir par exemple la demande de brevet publiée US20040061867 [Réf.3]). De plus, les éléments hors diagonaux renseignent sur le niveau d'aberration et le niveau de diffusion multiple.

A partir de cette matrice de réflexion dans le plan focal, une nouvelle approche matricielle pour l'imagerie optique permettant la correction simultanée des aberrations sur plusieurs domaines d'isoplanétisme du champ de vision a été décrite. Voir par exemple US20210310787 [Réf. 4]. Dans [Réf. 4], une nouvelle matrice, dite « *matrice de distorsion* », a été introduite pour la caractérisation de milieux hétérogènes. Cette matrice contient uniquement la partie distordue des fronts d'onde rétrodiffusés par l'échantillon pour un ensemble de points de focalisation $\mathbf{r_{in}}$. Cette matrice permet de révéler les corrélations spatiales du champ lumineux liées à l'isoplanétisme. L'analyse des corrélations entre ces différents fronts d'onde permet d'extraire les lois d'aberrations locales sur l'ensemble du champ de vision. Il est ainsi possible d'accéder aux matrices de transmission entre le plan focal objet au sein de l'échantillon et les plans source et image situés à l'extérieur de ce dernier. Une fois conjuguées en phase ou inversées, ces matrices fournissent l'ensemble des lois de focalisation permettant de corriger de manière optimale et localement les aberrations en chaque point du champ de vision.

La méthode de caractérisation décrite dans [Réf. 4] est cependant limitée à l'imagerie d'un seul plan transverse dit plan de cohérence dans l'échantillon. Pour accéder à une image tridimensionnelle de l'échantillon, il faudrait scanner axialement l'échantillon. Un scan axial limite la vitesse d'acquisition et donc le volume accessible, notamment pour des applications in-vivo ou pour l'imagerie dynamique de tissus cellulaires par exemple.

Surtout, les déposants ont montré que la méthode de caractérisation décrite dans [Réf. 4] ne permet qu'une correction transverse des aberrations pour un plan de cohérence dont la position est dictée par celle du miroir de référence dans le montage interférométrique. Or les inhomogénéités d'indice optique dans le milieu induisent un déplacement axial et une déformation du volume de cohérence par rapport à sa position et sa planéité qui seraient attendues si l'indice optique du milieu était homogène. Autrement dit, le plan focal objet de l'objectif de microscope et le plan de cohérence ne sont plus confondus en cas d'inhomogénéités d'indice optique dans le milieu. Cela limite le rapport signal-à-bruit, génère de fortes aberrations transverses ainsi que des distorsions axiales sur l'image.

La présente description propose des procédés et systèmes de caractérisation optique d'un milieu volumique et diffusant, également basée sur une approche matricielle, permettant une caractérisation ultra-rapide en volume de l'échantillon, typiquement inférieure à une seconde, et permettant en outre d'accéder à des images de réflectivité dont la dimension axiale n'est plus dictée par le temps de vol des photons diffusés mais par la profondeur réelle des diffuseurs dans l'échantillon.

## Résumé

La présente description a pour objet, selon un premier aspect, un procédé de caractérisation optique d'un échantillon formé d'un milieu volumique et diffusant, le procédé comprenant :

- une étape de positionnement dudit échantillon dans un champ de vision d'un premier objectif de microscope, ledit objectif de microscope étant situé dans un bras objet d'un premier interféromètre, ledit premier interféromètre comprenant en outre un bras de référence;
- une étape de génération, au moyen d'un dispositif d'illumination comprenant une source lumineuse à large bande spectrale, d'une première pluralité de $N_{in}$ ondes lumineuses incidentes présentant des fronts d'onde différents ;
- pour chaque onde lumineuse incidente de front d'onde donné, une étape d'acquisition au moyen d'un détecteur comprenant $N_{out}$ détecteurs élémentaires, d'une deuxième pluralité de signaux d'interférence, chaque signal d'interférence résultant de l'interférence, sur un plan de détection du détecteur, entre une onde rétrodiffusée par l'échantillon illuminé par ladite onde lumineuse incidente, et une onde de référence issue du bras de référence, lesdits signaux d'interférence étant acquis pour $N_{\omega}$ fréquences différentes ou $N_{\omega}$ différences de marche différentes entre l'onde rétrodiffusée et l'onde de référence;

- la détermination d'une matrice réflexion polychromatique tridimensionnelle $\tilde{\mathbf{R}}_{\rho u} = [\tilde{R}(\rho_{\mathbf{out}}, \mathbf{u_{in}}, \omega)]$ de taille $N_{in}$ x $N_{out}$ x $N_{\omega}$, ladite matrice réflexion polychromatique tridimensionnelle comprenant l'ensemble des signaux d'interférence acquis pour les $N_{in}$ ondes lumineuses incidentes et $N_{\omega}$ fréquences;
- la détermination numérique, par application d'un premier propagateur, à partir de ladite matrice réflexion polychromatique, d'au moins une première matrice de réflexion volumique focalisée, comprenant un ensemble de réponses de l'échantillon entre des points sources et des points de réception conjugués avec des voxels $\mathbf{r_{in}}(x'_{in}, y'_{in}, z_{in})$ et $\mathbf{r_{out}}$ $(x'_{out}, y'_{out}, z_{out})$ de l'échantillon situés respectivement à des profondeurs $z_{in}$ et $z_{out}$ dans l'échantillon;
- la détermination, à partir de ladite première matrice de réflexion volumique focalisée, d'au moins une cartographie d'un paramètre physique dudit échantillon.

Dans la présente description, le champ de vision de l'objectif de microscope comprend un plan focal objet dudit objectif. Les dimensions du champ de vision sont limitées par les caractéristiques de l'objectif de microscope, les dimensions de la surface de détection et le grandissement d'un dispositif d'imagerie entre le plan focal objet et la surface de détection. Les dimensions du champ de vision peuvent être également ajustées en fonction de l'étendue transverse de l'échantillon que l'opérateur souhaite caractériser.

Dans la présente description, on appelle source à large bande spectrale une source dont la largeur de la bande spectrale $\Delta\omega$ est égale ou supérieure à une valeur $\Delta\omega_{min}$ permettant d'obtenir une résolution axiale $\delta z$ souhaité pour la détermination de la cartographie des paramètres physiques : $\delta z = 2\pi c_0/(\overline{n}\Delta\omega_{min})$, où $\overline{n}$ est l'indice optique moyen de l'échantillon et $c_0$ est vitesse de la lumière. Selon des exemples de réalisation, la largeur de bande spectrale de la source est comprise entre environ 10 nm et environ 400 nm. La source est par exemple, mais non limitativement, une source à balayage spectral ou une source à large bande spectrale, par exemple une diode électroluminescente (LED), une source halogène, un laser femtoseconde, une diode superluminescente.

Les déposants ont montré que la détermination de la matrice de réflexion volumique focalisée, obtenue à partir de la matrice réflexion polychromatique, permet de s'affranchir des distorsions axiales et transverses inhérentes aux techniques décrites dans l'état de l'art [Réf. 4].

Il devient en effet possible, pour former par exemple une image confocale avec une résolution seulement limitée par la diffraction, de faire coïncider le volume de cohérence avec le plan de focalisation géométrique.

Une cartographie d'un paramètre physique de l'échantillon est comprise dans la présente description et sans précision contraire comme une cartographie plane ou volumique dudit paramètre.

Ainsi, une cartographie d'un paramètre physique de l'échantillon comprend selon un ou plusieurs exemples de réalisation une image confocale en réflexion, *en face* ou volumique, une cartographie de la fonction d'étalement de l'énergie en réflexion (ou RPSF pour « *reflective point spread function* ») autour de voxels de l'échantillon, une cartographie du taux de diffusion simple, plane ou volumique, une cartographie du taux de diffusion multiple, plane ou volumique, une cartographie de l'indice optique de l'échantillon, plane ou volumique.

Une image confocale en réflexion *en face* est un estimateur de la réflectivité d'une section transverse de l'échantillon. Une image confocale volumique en réflexion est un estimateur de la réflectivité d'un volume de l'échantillon. Dans la suite de la description, on pourra appeler une image confocale en réflexion simplement « *image confocale* ».

Un voxel est défini comme une cellule volumique de résolution. Dans la présente description, un voxel peut également être nommé par son barycentre appelé « *point de focalisation* ». Les dimensions d'une cellule volumique de résolution sont déterminées par la résolution transverse d'un système optique défini par l'ensemble des éléments optiques situés entre l'échantillon et les plans d'illumination et de détection et la résolution axiale $\delta z$.

Dans la suite de la description, on appellera plus simplement la fonction de l'étalement de l'énergie en réflexion autour d'un point de focalisation, « *fonction d'étalement autour d'un point de focalisation* » ou « *RPSF audit point de focalisation* ». On appellera « *cartographie de la fonction d'étalement* » une cartographie de la fonction de l'étalement de l'énergie en réflexion (RPSF) autour d'une pluralité de points de focalisation de l'échantillon.

Selon un ou plusieurs exemples de réalisation, le procédé de caractérisation optique selon le premier aspect comprend en outre :

- la détermination à partir de ladite matrice de réflexion volumique focalisée d'une première image confocale en réflexion;
- la détermination, à partir de ladite matrice de réflexion volumique focalisée, d'une cartographie de la fonction d'étalement et la détermination d'une cartographie de la position du maximum d'intensité de chaque fonction étalement;
- la détermination à partir de la première image confocale en réflexion et de ladite cartographie de la position du maximum d'intensité, d'une deuxième image confocale en réflexion corrigée d'un défaut d'alignement et/ou de mise au point du premier interféromètre. Ainsi, les déposants ont mis en évidence un premier avantage de la détermination de la matrice de réflexion volumique focalisée, pour la détermination d'images confocales corrigées des défauts d'alignement et/ou de mise au point du premier interféromètre.

Selon un ou plusieurs exemples de réalisation, le procédé de caractérisation optique selon le premier aspect comprend en outre :

- à partir de ladite première matrice de réflexion volumique focalisée, la détermination d'une pluralité de cartographies de la fonction d'étalement pour une pluralité de valeurs d'un indice optique intégré de l'échantillon
  _ à partir de ladite pluralité de cartographies de la fonction d'étalement, la détermination d'une pluralité de cartographies de la position du maximum d'intensité de la fonction d'étalement, chaque cartographie étant obtenue pour une valeur de l'indice optique intégré ;
- la détermination d'une cartographie de la valeur optimale de l'indice optique intégré pour laquelle, en chaque point de focalisation, la valeur maximale d'intensité de la fonction d'étalement est la plus grande;
- la détermination numérique d'un deuxième propagateur basé sur ladite cartographie de la valeur optimale de l'indice optique intégré; et

la détermination d'une deuxième matrice de réflexion volumique focalisée utilisant ledit deuxième propagateur.

Dans la présente description, on appelle indice optique intégré de l'échantillon, l'indice optique de l'échantillon intégré entre une surface proximale de ce dernier (c'est-à-dire du côté de l' objectif de microscope) et le point de focalisation considéré.

Selon un ou plusieurs exemples de réalisation, le procédé comprend en outre, à partir de ladite cartographie de la valeur optimale de l'indice optique intégré, la détermination d'une cartographie de l'indice optique du milieu en chaque point de focalisation. Ledit deuxième propagateur peut alors être déterminé sur la base de ladite cartographie de l'indice optique. Ainsi, selon un ou plusieurs exemples de réalisation, le procédé de caractérisation optique selon le premier aspect comprend en outre :

- à partir de ladite première matrice de réflexion volumique focalisée, la détermination d'une pluralité de cartographies de la fonction d'étalement pour une pluralité de valeurs d'un indice optique intégré de l'échantillon ;
  _ à partir de ladite pluralité de cartographies de la fonction d'étalement, la détermination d'une pluralité de cartographies de la position du maximum d'intensité de la fonction d'étalement, chaque cartographie étant obtenue pour une valeur de l'indice optique intégré ;
- la détermination d'une cartographie de la valeur optimale de l'indice optique intégré pour laquelle, en chaque point de focalisation, la valeur maximale d'intensité de la fonction d'étalement est la plus grande;
- à partir de ladite cartographie de la valeur optimale de l'indice optique intégré, la détermination d'une cartographie de l'indice optique du milieu en chaque point de focalisation ;
- la détermination numérique d'un deuxième propagateur basé sur ladite cartographie de l'indice optique du milieu; et

la détermination d'une deuxième matrice de réflexion volumique focalisée utilisant ledit deuxième propagateur.

Ledit deuxième propagateur, qu'il soit optimisé à partir de l'indice intégré directement ou à partir de l'indice de l'échantillon dérivé de l'indice intégré pour rendre maximale l'intensité de la RPSF au point de focalisation, correspond à un propagateur qui permet de faire coïncider sensiblement volume de cohérence et plan de focalisation géométrique en prenant en compte des inhomogénéités d'indice dans le milieu volumique et diffusant.

A partir de la deuxième matrice de réflexion volumique focalisée qui en résulte, il est possible d'établir une image confocale de la réflectivité du milieu, image dont on a corrigé les défauts de mis au point et distorsion axiales dont souffrait l'image confocale initiale. Une cartographie de la RPSF associée à cette seconde matrice de réflexion volumique focalisée peut par ailleurs permettre de quantifier localement un taux de diffusion multiple pour chaque voxel de l'image. Ce paramètre nous donne un indice de fiabilité local de l'image et caractérise localement les propriétés de diffusion du milieu. Ladite cartographie de la RPSF permet également de quantifier un taux de diffusion simple dont le gradient en profondeur donne accès à une estimation locale du libre parcours moyen de diffusion.

Selon un ou plusieurs exemples de réalisation, le procédé comprend en outre la détermination, à partir de la deuxième matrice de réflexion volumique focalisée, d'une matrice distorsion définie entre une base de correction et une base focalisée, la détermination de la matrice distorsion comprenant :

- la projection, en entrée ou en sortie, de la deuxième matrice de réflexion volumique focalisée, dans la base de correction, pour obtenir une matrice de réflexion projetée respectivement en entrée ou en sortie;
- une détermination de la matrice distorsion par produit terme à terme entre ladite matrice de réflexion projetée et une matrice de réflexion de référence, définie pour un milieu de référence, dans ladite base de correction;
- une détermination locale des invariants de ladite matrice distorsion, afin d'identifier, dans la base de correction, des lois d'aberration dans des sous domaines du champ de vision ;
- l'estimation à partir desdites lois d'aberration, d'une matrice de transmission entre des voxels du champ de vision et la base de correction.

Une base focalisée, respectivement en entrée ou en sortie, est l'ensemble des points source, respectivement points de réception, conjugués aux voxels pavant l'échantillon.

Une correction locale des aberrations ainsi réalisée permet d'obtenir une image confocale dont le contraste et la résolution sont optimisées dans tout le volume de l'échantillon.

Selon un ou plusieurs exemples de réalisation, la matrice distorsion est déterminée de la même manière mais à partir de la première matrice de réflexion volumique focalisée, obtenue au moyen du premier propagateur lorsqu'il n'y a pas eu d'étape d'optimisation du propagateur pour rendre maximale l'intensité de la fonction d'étalement en chaque point de focalisation.

Un produit terme à terme entre les matrices est également connu sous le nom de produit d'Hadamard.

Selon un ou plusieurs exemples de réalisation, le milieu de référence est un milieu homogène d'indice optique égal à l'indice moyen du milieu de propagation. La matrice de réflexion de référence peut être établie théoriquement pour ce milieu de référence avec un miroir plan dans le plan focal de l'objectif de microscope. Suivant le degré de connaissance *a priori* du milieu de propagation, le milieu de référence pourra prendre des formes plus élaborées (*e.g* milieu multicouches *etc*.). Dans ce cas, la matrice de référence pourra être calculée numériquement. La construction de la matrice distorsion revient à soustraire à la phase de chaque élément de la matrice de réflexion projetée, la phase de l'élément correspondant de la matrice de réflexion de référence.

Selon un ou plusieurs exemples de réalisation, la base de correction dans laquelle est définie ladite matrice distorsion est une base maximisant la taille des domaines d'isoplanétisme contenus dans le champ de vision, par exemple l'ensemble des points d'un plan conjugué avec le plan d'un aberrateur si celui-ci est bidimensionnel, ou par exemple un plan conjugué avec le plan pupille de l'objectif de microscope.

Selon un ou plusieurs exemples de réalisation, une détermination locale des invariants de ladite matrice distorsion comprend, comme décrit dans [Réf. 4], une décomposition en valeurs singulières de ladite matrice distorsion, une décomposition en valeurs singulières d'une matrice distorsion normalisée, c'est à dire dont le module de chacun des éléments aura été normalisé mais dont la phase aura été préservée, une décomposition en valeurs singulières d'une matrice de corrélation normalisée de ladite matrice distorsion, c'est-à-dire une matrice de corrélation de ladite première matrice distorsion dont le module de chacun des éléments aura été normalisé. D'autres méthodes sont possibles pour la détermination locale des invariants, comme par exemple un processus itératif de retournement de phase appliqué à ladite matrice distorsion.

Selon un ou plusieurs exemples de réalisation, la source lumineuse est une source à longueur d'onde variable et pour chaque onde lumineuse incidente de front d'onde donné, les signaux d'interférence de la deuxième pluralité $N_\omega$ de

signaux d'interférence sont acquis pour $N_\omega$ fréquences (ou longueurs d'onde) de ladite onde lumineuse incidente et une différence de marche fixe entre le bras objet et le bras de référence du premier interféromètre. Par fréquence d'une onde incidente (respectivement longueur d'onde), on comprend dans la présente description la fréquence centrale (respectivement longueur d'onde centrale) du spectre de l'onde incidente.

Selon un ou plusieurs exemples de réalisation, pour chaque onde lumineuse incidente de front d'onde prédéterminé, les signaux d'interférence de ladite deuxième pluralité de signaux d'interférence sont acquis pour $N_\omega$ différences de marche entre le bras objet et le bras de référence du premier interféromètre. Une simple opération de transformée de Fourier temporelle permet alors d'obtenir, à partir desdits signaux d'interférence acquis pour des différences de marche, ou temps de vol, différentes, la matrice réflexion polychromatique. Selon un ou plusieurs exemples de réalisation, la matrice réflexion polychromatique est générée entre une base de mesure à l'émission qui est une base des ondes planes (plan pupille entrée) et une base de mesure à la réception (plan de détection). Un propagateur permettant de déterminer une première matrice de réflexion volumique focalisée à partir de ladite matrice réflexion polychromatique, comprend par exemple un premier changement de base à l'entrée qui permet de passer du plan pupille entrée à un plan de focalisation à l'émission, et un deuxième changement de base à la sortie qui permet de passer du plan de détection à un plan de focalisation à la réception, aussi appelé plan de focalisation en sortie. De tels propagateurs sont connus de l'homme du métier. Ces propagateurs peuvent, par exemple, comprendre des opérations de transformées de Fresnel.

Selon un ou plusieurs exemples de réalisation, les $N_{in}$ ondes lumineuses incidentes sont cohérentes spatialement et présentent des fronts d'onde contrôlés par des moyens de mise en forme spatiale du front d'onde.

Par exemple, les moyens de mise en forme spatiale du front d'onde comprennent des moyens de balayage et $N_{in}$ ondes lumineuses incidentes sont des ondes planes présentant chacune un vecteur d'onde avec une direction différente.

Les fronts d'onde prédéterminés ne sont cependant pas nécessairement des ondes planes avec des vecteurs d'onde présentant des directions différentes. Par exemple, le dispositif d'illumination peut comprendre un modulateur spatial de front d'onde, par exemple un miroir déformable ou un modulateur à cristaux liquides, pour générer des fronts d'onde prédéterminés différents.

Des ondes lumineuses incidentes sont cohérentes spatialement quand la fonction de cohérence mutuelle du champ électromagnétique est uniforme sur un plan de l'espace.

A contrario, une onde lumineuse est incohérente spatialement quand la fonction de cohérence mutuelle du champ électromagnétique a un support seulement limité par la diffraction, i.e de l'ordre de la demi-longueur d'onde.

Dans la présente description, on appelle onde partiellement cohérente spatialement une onde dont la fonction de cohérence mutuelle présente un support fini dont la largeur est supérieure à la demi-longueur d'onde.

Selon un ou plusieurs exemples de réalisation, ladite source lumineuse pour l'émission d'ondes lumineuses incidentes cohérentes spatialement est un laser monomode à balayage spectral, par exemple un laser émettant dans une plage spectrale comprise entre des longueurs d'onde égales à environ 800 et environ 875 nm.

Selon un ou plusieurs exemples de réalisation, ladite source lumineuse est une source à faible cohérence spatiale, le procédé comprenant en outre :

- la génération, à partir de chaque onde lumineuse incohérente spatialement ou partiellement cohérente issue de la source lumineuse, et au moyen d'un deuxième interféromètre, de deux ondes d'illumination polarisées de polarisations orthogonales et présentant un décalage spatial dans un plan conjugué avec un plan focal du premier objectif de microscope;
- la variation dudit décalage spatial pour générer les $N_{in}$ ondes incidentes de front d'onde différents ;
- l'envoi, pour chaque décalage spatial, desdites ondes polarisées de polarisations orthogonales respectivement sur les bras objet et de référence dudit premier interféromètre au moyen d'un élément séparateur de polarisation;

- l'acquisition au moyen du détecteur, de ladite deuxième pluralité de signaux d'interférence, chaque signal d'interférence résultant de l'interférence, sur le plan de détection du détecteur, entre une onde rétrodiffusée par l'échantillon illuminé par l'une desdites ondes polarisées de polarisations orthogonales, et une onde de référence générée par la réflexion de l'autre desdites ondes polarisées de polarisations orthogonales par un miroir de référence du bras de référence, lesdits signaux d'interférence étant acquis pour $N_\omega$ différences de marche différentes entre l'onde rétrodiffusée et l'onde de référence ;
- ladite matrice réflexion polychromatique étant déterminée à partir de l'ensemble des signaux d'interférence acquis pour les $N_{in}$ décalages spatiaux et $N_\omega$ différences de marche.

Ladite source à faible cohérence spatiale est par exemple une diode électroluminescente ou une lampe halogène.

Le procédé de caractérisation ainsi décrit présente l'avantage d'être plus rapide, en termes de temps d'acquisition, et optimal, en termes de rapport signal-à-bruit, pour l'obtention d'une image confocale d'une ou plusieurs sections transverses de l'échantillon.

La présente description a pour objet, selon un deuxième aspect, un système de caractérisation optique pour la mise en œuvre de procédés de caractérisation optique d'un échantillon conformes au premier aspect.

Ainsi, la présente description a pour objet un système de caractérisation optique d'un échantillon formé d'un milieu volumique et diffusant, comprenant :

- un premier interféromètre avec un bras objet et un bras de référence, le bras objet comprenant un premier objectif de microscope avec un champ de vision donné dans lequel est positionné, en opération, l'échantillon;
- un dispositif d'illumination comprenant une source lumineuse à large bande spectrale, configuré pour générer une première pluralité d'ondes lumineuses incidentes présentant des fronts d'onde différents ;
- un détecteur comprenant $N_{out}$ détecteurs élémentaires, configuré pour l'acquisition, pour chaque onde lumineuse incidente de front d'onde donné, d'une deuxième pluralité de signaux d'interférence, chaque signal d'interférence résultant de l'interférence, sur un plan de détection du détecteur, entre une onde rétrodiffusée par l'échantillon illuminé par ladite onde lumineuse incidente, et une onde de référence issue du bras de référence, lesdits signaux d'interférence étant acquis pour $N_{\omega}$ fréquences différentes ou $N_{\omega}$ différences de marche différentes entre l'onde rétrodiffusée et l'onde de référence;

- une unité de traitement configurée pour :
- la détermination d'une matrice réflexion polychromatique tridimensionnelle $\tilde{\mathbf{R}}_{\rho u} = [\tilde{R}(\rho_{\mathbf{out}}, \mathbf{u_{in}}, \omega)]$ de taille $N_{in}$ x $N_{out}$ x $N_{\omega}$, ladite matrice réflexion polychromatique tridimensionnelle comprenant l'ensemble des signaux d'interférence acquis pour les $N_{in}$ ondes lumineuses incidentes et $N_{\omega}$ fréquences;
- la détermination numérique, par application d'un premier propagateur, à partir de ladite matrice réflexion polychromatique, d'au moins une première matrice de réflexion volumique focalisée comprenant un ensemble de réponses de l'échantillon entre des points sources et des points de réception conjugués avec des voxels $\mathbf{r_{in}}(x'_{in}, y'_{in}, z_{in})$ et $\mathbf{r_{out}}(x'_{out}, y'_{out}, z_{out})$ de l'échantillon situés respectivement à des profondeurs $z_{in}$ et $z_{out}$ dans l'échantillon;
- la détermination, à partir de ladite première matrice de réflexion volumique focalisée, d'au moins une cartographie d'un paramètre physique dudit échantillon.

Selon un ou plusieurs exemples de réalisation, ledit premier interféromètre est un interféromètre de Linnik et le bras de référence comprend un miroir de référence et un deuxième objectif de microscope.

Cependant, d'autres arrangements sont possibles pour le premier interféromètre que l'homme du métier pourra mettre en œuvre avec ses connaissances générales. Notamment, le premier interféromètre peut ne pas comprendre de miroir de référence et/ou d'objectif de microscope sur le bras de référence.

Selon un ou plusieurs exemples de réalisation, le détecteur comprend un dispositif d'acquisition bidimensionnelle, par exemple une caméra de type CCD ou CMOS.

Dans d'autres exemples de réalisation, le détecteur comprend un spectromètre. L'acquisition des signaux d'interférence pour $N_{\omega}$ fréquences peut alors être fait à la détection.

Selon un ou plusieurs exemples de réalisation la source lumineuse est une source à faible cohérence spatiale; et le dispositif d'illumination comprend :

- un deuxième interféromètre configuré pour générer à partir de chaque onde lumineuse incohérente spatialement ou partiellement cohérente issue de la source lumineuse, deux ondes d'illumination polarisées de polarisations orthogonales et présentant un décalage spatial dans un plan conjugué avec un plan focal du premier objectif de microscope; et
- des moyens de variation du décalage spatial ; et dans lequel
- ledit premier interféromètre est un interféromètre de Linnik et comprend
- un élément séparateur de polarisation configuré pour envoyer respectivement sur

les bras objet et de référence, chacune desdites ondes polarisées de polarisations orthogonales et présentant ledit décalage spatial ;

- des moyens de variations de la différence de marche entre les bras objet bras de référence ; et dans lequel
- chaque signal d'interférence de ladite deuxième pluralité de signaux d'interférence résulte de l'interférence, sur le plan de détection du détecteur, entre une onde rétrodiffusée par l'échantillon illuminé par l'une desdites ondes polarisées de polarisations orthogonales, et une onde de référence générée par la réflexion de l'autre desdites ondes polarisées de polarisations orthogonales par un miroir de référence du bras de référence, lesdits signaux d'interférence étant acquis pour $N_{\omega}$ différences de marche différentes entre l'onde rétrodiffusée et l'onde de référence ;
- ladite matrice réflexion polychromatique est déterminée à partir de l'ensemble des signaux d'interférence acquis pour

les $N_{in}$ décalages spatiaux et $N_{\omega}$ différences de marche.

**Brève description des figures**

D'autres avantages et caractéristiques de la technique présentée ci-dessus apparaîtront à la lecture de la description détaillée ci-dessous, faite par référence aux figures dans lesquelles :

- La Fig. 1A, un premier exemple d'un système de caractérisation optique d'un milieu volumique et diffusant selon la présente description ;
- La Fig. 1B, un schéma illustrant les différentes notations utilisées pour les bases dans lesquelles sont décrites les matrices dans des procédés de caractérisation selon la présente description ;
- La Fig. 2A, un schéma illustrant le balayage par $N_{in}$ impulsions lumineuses de la pupille d'entrée d'un objectif de microscope agencé sur l'un et l'autre des bras de référence et bras objet d'un premier interféromètre dans un exemple d'un système de caractérisation tel qu'illustré sur la Fig. 1A, ;
- La Fig. 2B, un schéma illustrant la répartition en intensité d'un signal d'interférence sur un pan de détection d'un détecteur d'un système de caractérisation tel qu'illustré sur la Fig. 1A;
- La Fig. 2C, une représentation d'un exemple de matrice réflexion polychromatique comprenant l'ensemble des signaux d'interférence acquis pour $N_{in}$ ondes lumineuses incidentes et $N_{\omega}$ longueurs d'onde, ladite matrice étant obtenue au moyen d'un procédé de caractérisation selon la présente description ;
- La Fig. 3A, un schéma illustrant la réponse du milieu entre un point source d'une base de focalisation en entrée et un point de réception d'une base focalisée en sortie, pour illustrer un élément d'un exemple d'une matrice de réflexion volumique focalisée selon la présente description ;
- La Fig. 3B, des images illustrant des cartographies de paramètres physiques obtenues à partir de la matrice de réflexion volumique focalisée objet du procédé selon la présente description ;
- La Fig. 3C, un schéma illustrant la non-coïncidence des plans de focalisation et de cohérence, à l'origine d'une qualité dégradée dans les opérations de focalisation à l'émission et à la réception, pour la construction de la matrice volumique focalisée ;
- La Fig. 4A, des schémas illustrant des fonctions d'étalement (RPSF) obtenues avec un premier propagateur de Fresnel en des points de focalisation situés à différents plans de focalisation ;
- La Fig. 4B, un schéma montrant l'évolution du maximum d'intensité de la RPSF en fonction de la profondeur z des plans de focalisation ;
- La Fig. 5, des images montrant l'effet de la double focalisation numérique sur une image confocale d'un échantillon formé d'une mire ;
- La Fig. 6, une étude quantitative de la RPSF mesurée dans un échantillon formé d'une cornée, lors d'un procédé de caractérisation selon la présente description ;
- La Fig. 7A, un schéma illustrant la subdivision du champ de vision réalisée pour extraire les RPSFs locales ;
- La Fig. 7B, une image illustrant une cartographie du taux de diffusion multiple à la profondeur z=250 $\mu$m dans l'échantillon formé de la cornée ;
- La Fig. 7C, une image illustrant une carte des RPSFs locales à la profondeur z=250 $\mu$m dans l'échantillon formé de la cornée ;
- La Fig. 7D, des images montrant deux sections longitudinales (B-scans) de l'indice optique $n(x, y, z)$ estimé dans l'échantillon formé de la cornée au moyen d'un procédé de caractérisation selon la présente description ;
- La Fig. 8, des images illustrant le résultat d'un procédé de correction numérique des aberrations transverses dans l'échantillon formé de la cornée à la profondeur z=250 $\mu$m, au moyen d'un procédé de caractérisation selon la présente description ;
- La Fig. 9 illustre la phase d'une matrice de transmission au moyen d'un processus itératif de correction des aberrations dans un exemple de procédé selon la présente description et des images confocales de l'échantillon formé de la cornée à une profondeur z = 250 $\mu$m, ainsi que des B-Scans, avant et après correction numérique des aberrations ;
- La Fig. 10A, un autre exemple d'un système de caractérisation optique d'un milieu volumique et diffusant selon la présente description, dans lequel le premier interféromètre est un interféromètre autre qu'un interféromètre de Linnik ;
- La Fig. 10B, un autre exemple d'un système de caractérisation optique d'un milieu volumique et diffusant selon la présente description, similaire à celui de la Fig. 10A mais mettant en œuvre un détecteur de type spectromètre ;
- La Fig. 11, un autre exemple d'un système de caractérisation optique d'un milieu volumique et diffusant selon la présente description, pour la mise en œuvre d'un exemple de procédé selon la présente description dans lequel une matrice de réflexion

polychromatique dans le domaine temporel et dans une base focalisée, est enregistrée. Dans les différents modes de réalisation qui vont être décrits par référence aux figures, des éléments semblables ou identiques portent les mêmes références.

## Description détaillée

Dans la description détaillée qui suit, seuls certains modes de réalisation sont décrits en détails pour assurer la clarté de l'exposé mais ces exemples ne visent pas à limiter la portée générale des principes ressortant de la présente description.

Les différents modes de réalisation et aspects décrits dans la présente description peuvent être combinés ou simplifiés de multiples manières. En particulier, les étapes des différents procédés peuvent être répétées, interverties, exécutées en parallèle, sauf précision contraire. Lorsque dans la présente description, il est fait référence à des étapes de calcul ou traitement pour la mise en œuvre notamment d'étapes de procédés, il est entendu que chaque étape de calcul ou traitement peut être mis en œuvre par logiciel, hardware, firmware, microcode ou toute combinaison appropriée de ces technologies. Lorsqu'un logiciel est utilisé, chaque étape de calcul ou traitement peut être mise en œuvre par des instructions de programme d'ordinateur ou du code logiciel. Ces instructions peuvent être stockées ou transmises vers un support de stockage lisible par un ordinateur (ou unité de calcul) et/ou être exécutées par un ordinateur (ou unité de calcul) afin de mettre en œuvre ces étapes de calcul ou traitement.

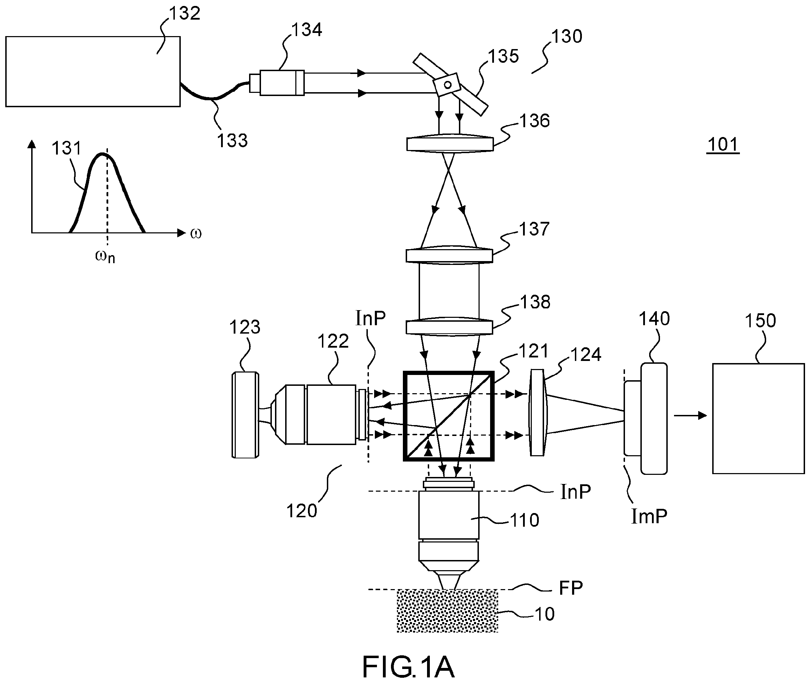

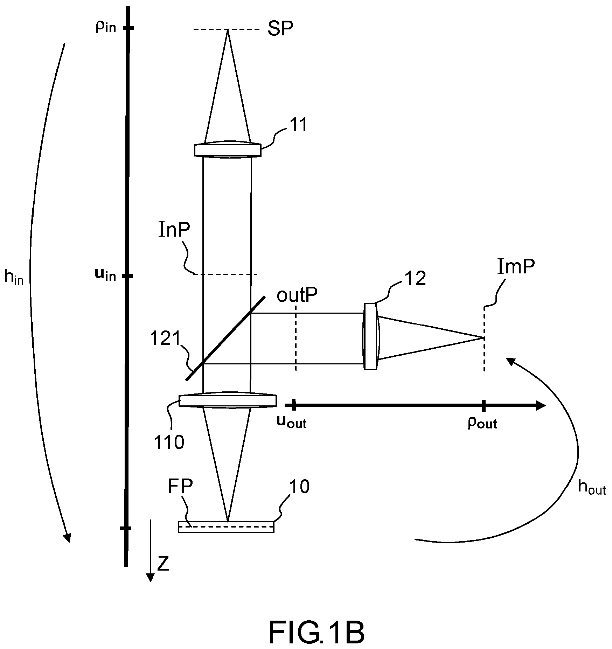

La Fig. 1A illustre un premier exemple d'un système 101 de caractérisation optique d'un échantillon 10 constitué d'un milieu volumique et diffusant, le système 101 étant configuré pour la mise en œuvre de procédés de caractérisation optique selon la présente description. La Fig. 1B représente un schéma illustrant de façon simplifiée les bases dans lesquelles sont décrites les matrices dans des procédés de caractérisation selon la présente description.

Le système 101 comprend un dispositif d'illumination 130 avec une source lumineuse 132 à large bande spectrale, par exemple une source à balayage spectral configurée pour l'émission d'un faisceau lumineux présentant une fréquence $\omega$ (ou longueur d'onde $\lambda$) qui peut varier sur une bande spectrale [$\omega_{min}$, $\omega_{max}$] (ou une plage de longueurs d'onde [$\lambda_{min}$, $\lambda_{max}$]), autour d'une fréquence centrale $\omega_n$ (ou longueur d'onde $\lambda_n$) comme cela est illustré schématiquement sur le spectre 131. La source lumineuse 132 émet par exemple dans le proche infra-rouge, par exemple entre environ $\lambda_{min}$ = 800 nm et environ $\lambda_{max}$ = 875 nm et peut balayer le spectre entre ces deux longueurs d'onde à une vitesse donnée, par exemple entre environ $10^2$ nm/s et environ $10^5$ nm/s. La source lumineuse est par exemple une source fibrée et le dispositif d'illumination peut comprendre une fibre de sortie 133 connectée à un collimateur 134 configuré pour l'émission d'un faisceau sensiblement collimaté, spatialement cohérent.

Le système 101 comprend par ailleurs un interféromètre 120 avec un bras objet et un bras de référence séparés par un cube séparateur 121. Le système 101 comprend un premier objectif de microscope 110 agencé dans le bras objet de l'interféromètre, l'échantillon étant positionné, en opération, dans un champ de vision de l'objectif de microscope.

Dans l'exemple illustré sur la Fig. 1A, l'interféromètre 120 est un interféromètre de Linnik comprenant, outre le premier objectif 110 sur le bras objet, un deuxième objectif de microscope 122 agencé dans le bras de référence. Le bras de référence comprend par ailleurs, dans cet exemple, un miroir de référence 123.

Comme illustré sur la Fig. 1A, le dispositif d'émission lumineuse comprend également, dans cet exemple, des moyens de balayage 135 du faisceau collimaté ainsi qu'un ensemble de lentilles 136, 137, 138. Les lentilles 136 et 137, appelées respectivement lentille de scan et lentille tube, forment un montage *4f* de grandissement donné, tandis que la lentille 138 est configurée pour focaliser le faisceau en sortie du montage *4f* dans un plan InP correspondant à un plan d'une pupille d'entrée de l'objectif de microscope 110 (« plan pupille entrée »). Les moyens de balayage 135 comprennent par exemple deux miroirs de balayage entraînés en rotation par des moteurs galvanométriques pour balayer le faisceau lumineux incident (indiqué avec des flèches simples sur la Fig. 1A) dans le plan pupille entrée InP de l'objectif de microscope 110 agencé sur le bras objet de l'interféromètre 120, dans les deux directions de l'espace. A noter que dans l'exemple de la Fig. 1A, du fait de l'utilisation d'un interféromètre de Linnik, le faisceau incident balaye également le plan pupille entrée du deuxième objectif de microscope 122 agencé sur le bras de référence de l'interféromètre. Le système 101 comprend par ailleurs un détecteur 140 avec un plan de détection ImP et une unité de traitement 150 configurée pour le traitement des données générées par le détecteur, comme cela sera expliqué par la suite. Le détecteur comprend par exemple une caméra configurée pour l'acquisition d'images bidimensionnelles avec une fréquence d'acquisition donnée. Par exemple la caméra permet d'acquérir des images 256 × 256 pixels à une fréquence donnée, par exemple autour de 75 kHz.

Bien entendu, d'autres configurations sont possibles pour le système de caractérisation optique 101 que l'homme du métier pourra mettre en œuvre au moyen de ses connaissances générales dans le domaine. Par exemple, le système 101 peut être déployé en configuration polarisée. Dans ce cas, le cube séparateur est un cube séparateur de polarisation. Deux lames quart-d'onde peuvent être placées entre les objectifs de microscope et le cube séparateur. En amont du détecteur 140, un analyseur de polarisation peut être agencé pour recombiner les deux polarisations.

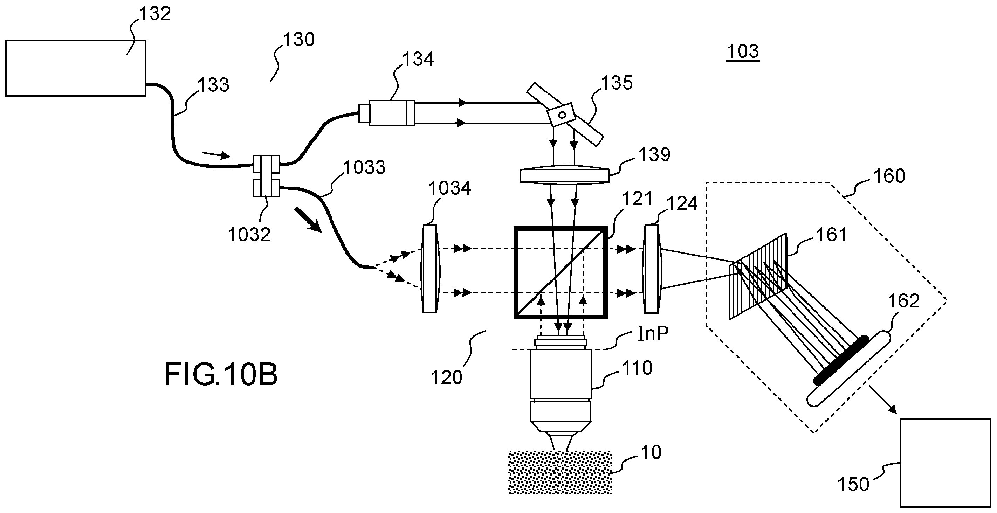

Par ailleurs, la source large bande n'est pas nécessairement à fréquence variable. Il est possible par exemple de remplacer la caméra par un spectromètre, comme cela sera décrit au moyen de la Fig. 10B, la sélection en fréquence se faisant à la détection.

Par ailleurs, plutôt que de faire varier la fréquence de la source, il est possible de façon équivalente de faire varier la différence de marche (ou « temps de vol ») entre les bras objet et de référence de l'interféromètre, par exemple en déplaçant le bloc constitué du miroir de référence 123 et du deuxième objectif de microscope 122.

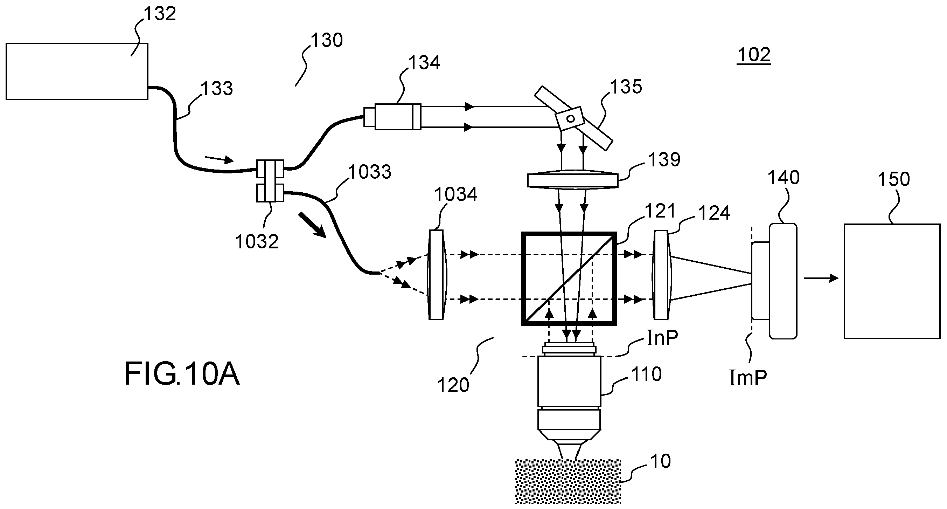

Par ailleurs, d'autres arrangements interférométriques sont possibles à la place de l'interféromètre de Linnik. Par exemple, comme cela sera décrit en référence à la Fig. 10A, l'onde de référence dans l'interféromètre peut provenir directement de la source.

Les notations utilisées dans la présente description pour identifier les différentes bases dans lesquelles sont décrites notamment les matrices de réflexion pour la caractérisation de l'échantillon sont rappelées au moyen de la Fig. 1B. La Fig. 1B n'illustre que quelques-uns des éléments du système de caractérisation par souci de simplification. Les notations s'appliquent au montage de la Fig. 1A mais, de façon plus générale, à tous les systèmes de caractérisation objet de la présente description.

Le plan focal de l'objectif de microscope 110 est référencé FP et est destiné à recevoir l'échantillon 10. On appelle base des ondes planes en entrée et on note InP le plan de la pupille d'entrée de l'objectif de microscope ou tout plan conjugué avec le plan de la pupille d'entrée de l'objectif de microscope. La pupille d'entrée est destinée à recevoir les ondes lumineuses incidentes pour l'illumination d'un champ de vision de l'échantillon que l'on cherche à caractériser. On note $\mathbf{u_{in}}$ un point du plan de la pupille d'entrée InP, défini par ses coordonnées cartésiennes $(v_{in}, w_{in})$. On appelle base des ondes planes en sortie et on note OutP le plan de la pupille de sortie de l'objectif de microscope ou tout plan conjugué avec le plan de la pupille de sortie de l'objectif de microscope. La pupille de sortie est destiné à recevoir les ondes lumineuses réfléchies par le champ de vision de l'échantillon que l'on cherche à caractériser. On note $\mathbf{u_{out}}$ un point du plan de la pupille de sorte OutP, défini par ses coordonnées cartésiennes $(v_{out}, w_{out})$. Comme illustré sur la Fig. 1B, les voies d'entrée et de sortie comprenant respectivement les pupilles d'entrée et de sortie sont séparées par l'élément séparateur de faisceau 121. Sur la voie d'entrée, on note SP le plan source du système optique, conjugué avec le plan focal de l'objectif de microscope, dans cet exemple symbolisé par une optique 11 formant avec l'objectif de microscope 110 un montage 4f. On note $\rho_{in}$ un point du plan source SP, défini par ses coordonnées cartésiennes $(x_{in}, y_{in})$. Sur la voie de sortie, on note ImP le plan image du système optique, conjugué avec le plan focal de l'objectif de microscope, dans cet exemple symbolisé par une optique 12 formant avec l'objectif de microscope 110 un montage 4f. On note $\rho_{out}$ un point du plan image ImP, défini par ses coordonnées cartésiennes $(x_{out}, y_{out})$.

Un voxel $\mathbf{r_{in}}$ de l'échantillon a pour coordonnées, $(\rho'_{in}, z_{in})$, où la coordonnée transverse $\rho'_{in}$ $(x'_{in}, y'_{in})$ du point $\mathbf{r_{in}}$ est conjugué d'un point $\rho_{in}$ du plan source SP. On appelle base focale en entrée la base (volumique) des points sources conjugués des voxels $\mathbf{r_{in}}$.

Un voxel $\mathbf{r_{out}}$ de l'échantillon a pour coordonnées, $(\rho'_{out}, z_{out})$, où la coordonnée transverse $\rho'_{out}$ $(x'_{out}, y'_{out})$ du point $\mathbf{r_{out}}$ est conjugué d'un point $\rho_{out}$ du plan image ImP. On appelle base focale en sortie la base (volumique) des points de réception conjugués des voxels $\mathbf{r_{out}}$. En opération, le dispositif d'illumination 130 permet de mettre en œuvre le procédé de caractérisation selon un exemple conforme à la présente description, de la manière suivante. Le dispositif d'illumination génère, pour une fréquence (ou longueur donnée) donnée, une première pluralité de $N_{in}$ ondes lumineuses incidentes consistant en des fronts d'onde prédéterminés différents. Par exemple, comme illustré sur la Fig. 1A, les $N_{in}$ ondes lumineuses incidentes sont $N_{in}$ ondes planes avec des vecteurs d'ondes de directions différentes contrôlées par les moyens de balayage 135. Par exemple, une lentille 124 (lentille de sortie) assure une conjugaison optique entre le plan focal objet FP de l'objectif de microscope 110 et le plan de détection ImP du détecteur 140. Ainsi, selon un exemple, en opération, les moyens de balayage 135 coopèrent avec le montage *4f* 136, 137 et la lentille de focalisation 138 pour balayer, dans les deux directions de l'espace, le plan pupille InP des objectifs de microscope 110 et 122 agencés respectivement sur les bras objet et bras de référence de l'interféromètre de Linnik 120. Les $N_{in}$ ondes lumineuses incidentes ainsi générées ont chacune un vecteur d'onde $\mathbf{k_{in}}$ dont la direction et la norme dépendent notamment de l'angle de balayage imposé par les moyens de balayage 135 et de la fréquence $\omega$ (ou longueur d'onde $\lambda$) de l'onde incidente.

Par ailleurs, pour chaque onde lumineuse incidente de front d'onde prédéterminé, le détecteur 140 acquière une deuxième pluralité de $N_\omega$ signaux d'interférence, chaque signal d'interférence résultant de l'interférence, sur le plan de détection ImP du détecteur, entre une onde rétrodiffusée par l'échantillon 10 illuminé par ladite onde lumineuse incidente, et une onde de référence issue du bras de référence, les $N_\omega$ signaux d'interférence étant acquis pour $N_\omega$ fréquences différentes. Par exemple, dans l'exemple de la Fig. 1A dans lequel le dispositif d'illumination 130 comprend une source lumineuse à balayage spectral, les $N_\omega$ signaux d'interférence sont acquis en faisant varier la longueur d'onde de la source lumineuse.

Les champs rétrodiffusés par les deux bras de l'interféromètre interfèrent au niveau du plan de détection ImP du détecteur 140. Le terme d'interférence entre les deux bras pour une fréquence $\omega$ de l'onde incidente correspond au produit entre le champ réfléchi par l'échantillon $E_s$ et le conjugué en phase $E_r^*$ du champ de référence issu du bras de référence selon l'équation :

[Math 2]

$$\tilde{R}(\boldsymbol{\rho_{out}}, \mathbf{u_{in}}, \omega) = E_s(\boldsymbol{\rho_{out}}, \mathbf{u_{in}}, \omega) \times E_r^*(\boldsymbol{\rho_{out}}, \mathbf{u_{in}}, \omega)$$

Où $\rho_{out}(x_{out}, y_{out})$ est un vecteur de coordonnées spatiales $(x_{out}, y_{out})$ dans le plan de détection ImP (plan de mesure à la réception) et $\mathbf{u_{in}}(v_{in}, w_{in})$ est un vecteur de coordonnées spatiales $(v_{in}, w_{in})$ dans le plan pupille InP de l'objectif de microscope 110 (plan de mesure à l'émission).

En opération, la lentille 138 permet de focaliser le faisceau incident dans le plan pupille des objectifs de microscope. La tache focale associée, de dimension $\delta u$, est uniquement limité par la diffraction (ouverture numérique du système en amont de l'interféromètre). Cette tache focale est convertie par l'objectif de microscope en une quasi onde plane éclairant l'échantillon sur un grand champ de vision. Ce dernier sera imagé, en partie, sur le plan de détection ImP.

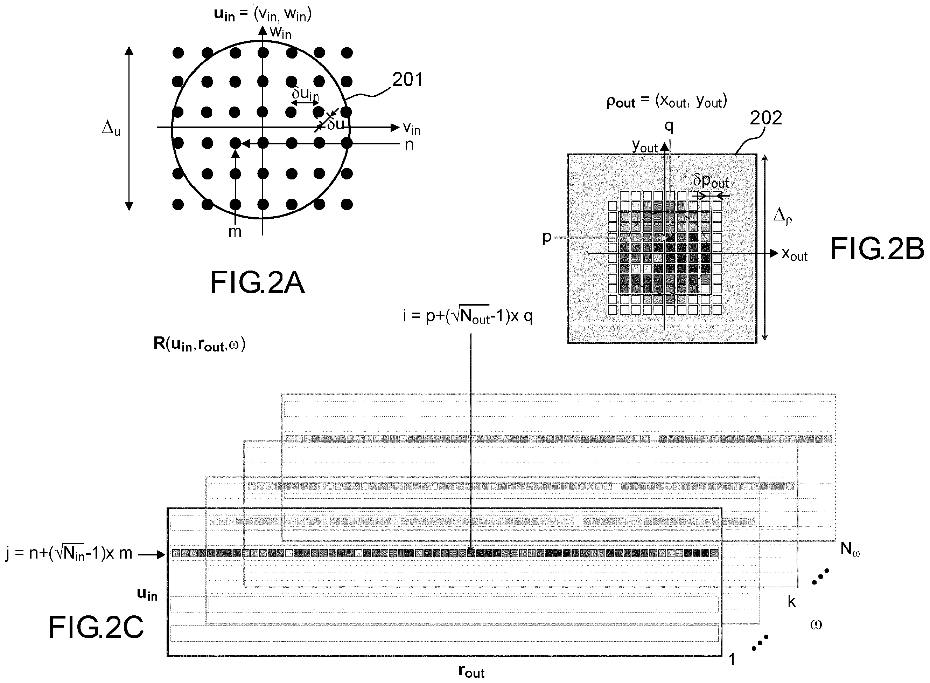

A titre d'illustration, la Fig. 2A illustre ainsi le balayage de la pupille d'entrée InP des objectifs de microscope 110, 122 par $N_{in}$ faisceaux lumineux incidents. Les miroirs de scan 135 permettent de balayer la pupille physique des objectifs de microscope dont la circonférence est représentée par un cercle noir 201 sur la Fig.2A. Les disques noirs désignent les positions de chacun des faisceaux incidents focalisés sur la pupille d'entrée InP des objectifs de microscope 110, 122. Le pas spatial entre chaque faisceau est noté $\delta u_{in}$. En tenant compte des caractéristiques des objectifs de microscope utilisés, la dimension caractéristique de chaque tache focale est par exemple de $\delta u = 8$ μm. Cela correspond à une illumination du champ de vision maximale de $\Delta r = \lambda f / \delta u = 2$ mm où $f$ est la distance focale de l'objectif de microscope 110. Le pas spatial, $\delta u_{in}$, correspond à l'échantillonnage du champ électromagnétique dans le plan pupille d'émission et indique la précision avec laquelle les fronts d'onde incidents seront résolus lorsqu'ils seront resynthétisés numériquement à partir de la matrice de réflexion mesurée. Ce pas spatial peut donc être avantageusement ajusté en fonction du niveau d'aberrations généré par le milieu ; on peut le choisir suffisamment fin pour capturer les fluctuations spatiales les plus rapides des distorsions subies par l'onde lumineuse lors de son passage dans le milieu. Suivant l'application visée, l'ensemble de la pupille d'entrée 201 ou seulement une sous-partie de cette dernière peut être balayée. Par exemple, pour pouvoir accéder à une image confocale hautement résolue, on pourra balayer l'ensemble de la pupille d'entrée comme représenté sur la Fig. 2A.

Pour chaque illumination de l'échantillon, le terme d'interférence $\tilde{R}(\rho_{out}, \mathbf{u_{in}}, \omega)$ est mesuré par un détecteur dans le plan de détection ImP.

La Fig. 2B illustre le champ de vision $\Delta\rho$ dans le plan de détection de la caméra et l'échantillonnage spatial $\delta\rho_{out}$ sous lequel est enregistré chaque signal d'interférence (ou interférogramme). On note $N_{out}$ le nombre de pixels (ou détecteurs élémentaires) de la caméra sur lesquels est acquis le signal d'interférence. Le champ de vision correspond à une image agrandie du plan focal de l'objectif de microscope d'un facteur G, G étant le grandissement d'un système d'imagerie composé de l'objectif de microscope et de la lentille de sortie 124. Dans l'exemple du système de la Fig. 1A, G=-$f_s/f_{om}$ avec $f_s$ et $f_{om}$ les focales des lentilles 124 et des objectifs de microscope 110 et 122.

L'ensemble des interférogrammes enregistrés pour chaque illumination $\mathbf{u_{in}}$ et chaque fréquence $\omega$ sont stockés sous la forme d'une matrice de réflexion polychromatique 3D, $\tilde{\mathbf{R}}_{\rho u} = [R(\rho_{out}, \mathbf{u_{in}}, \omega)]$, de taille $N_{out} \times N_{in} \times N_\omega$.

La Fig. 2C illustre selon un exemple une telle matrice de réflexion polychromatique 3D pour en montrer sa structure tri-dimensionnelle. La première dimension désigne le point de détection $\rho_{out}$ dans le plan de détection de la caméra, dont les coordonnées $(x_{out}, y_{out})$ sont repérées par l'indice $i = p + \left(\sqrt{N_{out}} - 1\right) \times q$. Avec $p = \left(\sqrt{N_{out}} + 1\right)/2 + (x_{out}/\delta\rho_{out})$ et $q = \left[\left(\sqrt{N_{out}} + 1\right)/2 + (y_{out}/\delta\rho_{out})\right]$. La seconde dimension désigne la coordonnée $\mathbf{u_{in}}$ de chaque faisceau incident focalisé dans le plan pupille d'entrée InP. Les coordonnées $(v_{in}, w_{in})$ de $\mathbf{u_{in}}$ sont repérées par l'indice $j = n + \left(\sqrt{N_{in}} - 1\right) \times m$. Avec $n = \left(\sqrt{N_{in}} + 1\right)/2 + (v_{in}/\delta u_{in})$ et $m = \left[\left(\sqrt{N_{in}} + 1\right)/2 + (y_{in}/\delta u_{in})\right]$. La troisième dimension, repérée par l'indice $k$, correspond à l'ensemble des fréquences $\omega$ auxquelles est enregistré le signal d'interférence.

Une durée totale théorique d'acquisition de la matrice $\tilde{\mathbf{R}}_{\rho u} = [\tilde{R}(\rho_{out}, \mathbf{u_{in}}, \omega)]$ peut donc être estimée, dans cet exemple, à $t_{acq} = N_{in}t_s$, où $t_s = N_\omega/f_c$ correspond à la durée d'acquisition d'une image du volume d'intérêt pour une seule illumination et $f_c$ est la fréquence d'acquisition de la caméra. Le nombre $N_\omega$ de fréquences (ou longueurs d'ondes) est ajusté en fonction de l'épaisseur L du milieu à imager. En effet, $N_\omega = \Delta\omega/\delta\omega$, avec $\Delta\omega = \omega_{max} - \omega_{min}$ la bande passante sur laquelle est mesurée $\tilde{\mathbf{R}}_{\rho u}$ et $\delta\omega$ l'échantillonnage fréquentiel dicté par l'épaisseur L du milieu à imager tel que $\delta\omega = c_0/(2\bar{n}L)$, avec $\bar{n}$ l'indice optique moyen dans l'échantillon et $c_0$ la vitesse de la lumière. A titre d'exemple, pour $N_{in} = 11^2$ ondes planes incidentes, $L = 500$ μm, et $f_c = 100$ kHz, la durée d'acquisition d'une image du volume d'intérêt est $t_s = 1$ ms et la durée totale d'acquisition de la matrice $\tilde{\mathbf{R}}_{\rho u}$ est $t_{acq} = 121$ ms. Une deuxième étape du procédé selon la présente description comprend la détermination numérique, par application d'un propagateur et à partir de ladite matrice réflexion polychromatique

$\tilde{\mathbf{R}}_{\rho\mathbf{u}}$ d'au moins une première matrice de réflexion volumique focalisée = $[R(\mathbf{r_{out}}, \mathbf{r_{in}}, \omega)]$. La matrice de réflexion volumique focalisée comprend un ensemble de réponses de l'échantillon entre des points sources et des points de réception conjugués avec des voxels du milieu volumique 10. Une réponse de l'échantillon à une fréquence donnée entre un point source et un point de détection est le coefficient de réflexion de l'échantillon, mesurée au point de détection quand une onde lumineuse à ladite fréquence est émise depuis le point source. La position des voxels est repérée par les vecteurs $\mathbf{r_{in}}$ = $(x'_{in}, y,_{in}, z_{in})$ à l'émission et $\mathbf{r_{out}}$ = $(x'_{out}, y'_{out}, z_{out})$ à la réception. Un voxel est défini comme une cellule volumique de résolution donnée. Par exemple, les dimensions d'une telle cellule volumique de résolution sont déterminées, dans l'exemple d'un système tel que représenté sur la Fig. 1A, par la résolution transverse du dispositif suivant (x, y) et la profondeur de champ ou la bande spectrale de la source suivant z. Contrairement à l'état de l'art [Ref.4], les voxels peuvent être situés à des profondeurs différentes dans le milieu, comme cela est expliqué ci-dessous. La profondeur z dans le milieu volumique 10 est définie selon un axe (z) parallèle à l'axe optique de l'objectif de microscope, par exemple par rapport à une origine $z_{ref}$ = 0 qui correspond à la différence de marche nulle entre les bras de référence et échantillon.

La détermination numérique de la matrice de réflexion volumique focalisée $\tilde{\mathbf{R}}_{\mathbf{rr}}$ à partir de la matrice réflexion polychromatique $\tilde{\mathbf{R}}_{\rho\mathbf{u}}$ comprend dans cet exemple un premier changement de base à l'entrée qui permet de passer du plan pupille entrée à la base focalisée à l'émission (base focalisée en entrée) et un deuxième changement de base à la sortie qui permet de passer du plan de détection à base de focalisation à la réception (base focalisée en sortie). Ces changements de base sont appelés dans la suite de la description opération de focalisation numérique à l'émission et à la réception ou opération de double focalisation.

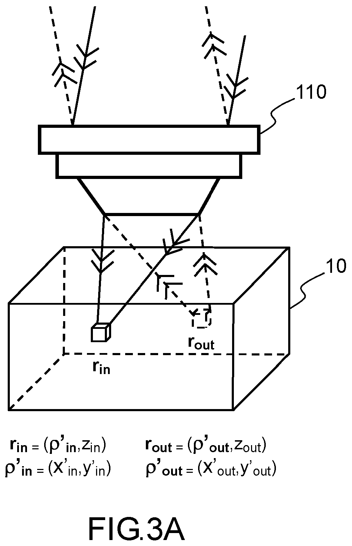

La Figure 3A illustre la procédure de focalisation numérique sur deux voxels situés aux points $\mathbf{r_{in}}$ et $\mathbf{r_{out}}$ situés à des profondeurs distinctes (respectivement $z_{in}$ et $z_{out}$) dans le milieu étudié.

Pour réaliser le passage de la matrice de réflexion polychromatique $\tilde{\mathbf{R}}_{\rho\mathbf{u}}$ à la matrice volumique focalisée $\tilde{\mathbf{R}}_{\mathbf{rr}}$, la double opération de focalisation à l'émission et à la réception est faite grâce à l'application, par exemple, d'un propagateur de Fresnel. A cette fin, la matrice de réflexion polychromatique $\tilde{\mathbf{R}}_{\rho\mathbf{u}}$ = $[\tilde{R}(\rho_{\mathbf{out}}, \mathbf{u_{in}}, \omega)]$ peut tout d'abord être projetée en sortie dans un plan de Fourier virtuel ( $\mathbf{u'_{out}}$ ) par simple transformée de Fourier spatiale sur la coordonnée de sortie $\rho_{\mathbf{out}}$ :

[Math 3]

$$\tilde{R}(\mathbf{u'_{out}}, \mathbf{u_{in}}, \omega) = \frac{1}{N_{out}} \sum_{\rho_{\mathbf{out}}} \tilde{R}(\rho_{\mathbf{out}}, \mathbf{u_{in}}, \omega) e^{-j\frac{2\pi}{\lambda f}\mathbf{u'_{out}} \cdot \rho_{\mathbf{out}}}$$

Des masques de Fresnel, notés $F(u, z_{in/out} - z_f, \omega)$, sont ensuite appliqués en entrée et sortie de la matrice résultante $\tilde{\mathbf{R}}_{\mathbf{uu}} = [\tilde{R}(\mathbf{u'_{out}}, \mathbf{u_{in}}, \omega)]$ pour translater numériquement les plans de focalisation d'émission et de réception, initialement situés à la profondeur $z_f$, à une profondeur $z_{in}$, respectivement $z_{out}$ dans l'échantillon, où $z_f$ est la distance focale de l'objectif de microscope 110 :

[Math 4]

$$\tilde{R}'(\mathbf{u'_{out}}, z_{out}, \mathbf{u_{in}}, z_{in}, \omega) = F(\mathbf{u'_{out}} - \mathbf{u_{in}}, z_{out} - z_f, \omega)\tilde{R}(\mathbf{u'_{out}}, \mathbf{u_{in}}, \omega)F(\mathbf{u_{in}}, z_{in} - z_f, \omega)$$

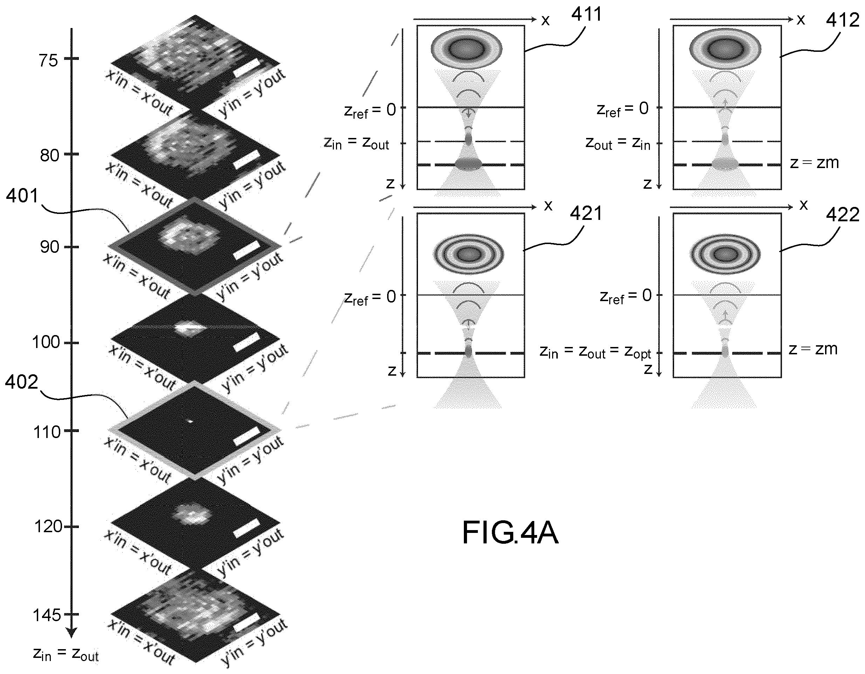

Des exemples de masque de Fresnel $F$, 411, 421, sont présentés sur la Fig. 4A pour différentes profondeurs $z = z_{in} = z_{out}$ de focalisation. Celui-ci s'exprime de la manière suivante :

[Math 5]

$$F(\mathbf{u}, z, \omega) = e^{j\left(\frac{2\pi\bar{n}}{\lambda} - k_z\right)z}$$

avec $\bar{n}$, l'indice optique supposé du milieu étudié considéré pour l'instant comme homogène et $k_z = \frac{2\pi}{\lambda}\sqrt{\bar{n}^2 - \frac{\|u\|^2}{f^2}}$,

le nombre d'onde axial. Une transformée de Fourier spatiale inverse peut être appliquée sur les coordonnées $\mathbf{u'_{out}}$ et $\mathbf{u_{in}}$ afin de projeter les champs incidents et réfléchis dans les bases focalisées $\mathbf{r_{out}}$ et $\mathbf{r_{in}}$ et ainsi obtenir la matrice de réflexion volumique focalisée $\tilde{\mathbf{R}}_{\mathbf{rr}}$ = $[R(\mathbf{r_{out}}, r_{in}, \omega)]$.

Une transformée de Fourier numérique sur les fréquences $\omega$ appliquée à la matrice réflexion focalisée polychromatique $\tilde{\mathbf{R}}_{\mathbf{rr}}$ permet alors de discriminer les photons diffusés suivant leur temps de vol dans l'échantillon. La matrice résultante est notée $\mathbf{R_{rr}} = [R(\mathbf{r_{out}}, \mathbf{r_{in}}, t)]$. Idéalement, i.e sous l'approximation de diffusion simple et pour un milieu d'indice $\bar{n}$ homogène, chaque temps de vol est associé à des photons diffusés à la profondeur $z = c_0 t/(2\bar{n})$ dans l'échantillon.

Pour illustrer l'intérêt de la matrice de réflexion focalisée volumique $\mathbf{R_{rr}}$, les déposants ont mesuré la matrice polychromatique $\tilde{\mathbf{R}}_{\rho u}$ à l'aide d'un système tel que décrit sur la Fig. 1A sur une cornée humaine opaque d'épaisseur $L = 400$ $\mu$m et d'indice moyen $\bar{n}$ = 1.4. L'ouverture numérique des objectifs de microscope est ON=0.3. Les paramètres d'acquisition sont les suivants : $N_{in}$ = 441, $N_\omega$ = 80, $\lambda_{min}$ =800 nm, $\lambda_{max}$ =875 nm, champ de vision dans le plan focal objet de l'objectif de microscope 716 $\times$ 716 $\mu$m.

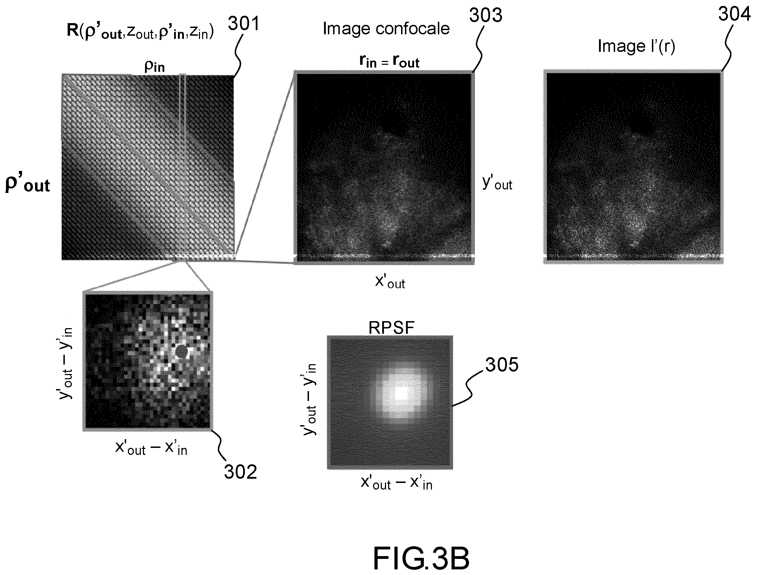

La Fig. 3B illustre ainsi selon un exemple une sous partie 301 (ou section transverse) de la matrice $\mathbf{R_{rr}} = [R(\mathbf{r_{out}}, \mathbf{r_{in}}, t)]$ construite à partir de $\tilde{\mathbf{R}}_{\rho u}$, pour $z = z_{in} = z_{out}$ = 250 $\mu$m et au temps balistique $t = 2\bar{n}z/c_0$ et une colonne 302 de cette sous-matrice réarrangée sous une forme bidimensionnelle.

Chaque colonne 302 de la sous partie 301 de la matrice $\mathbf{R_{rr}}$ donne le champ réfléchi mesuré par chaque point de réception virtuel $\mathbf{r_{out}}$ pour une onde lumineuse émise depuis le point source virtuel $\mathbf{r_{in}}$. Dans un cas idéal sans aberrations ni diffusion multiple, une tâche d'Airy serait observée autour du point source $\mathbf{r_{in}}$. Ici, au contraire, le champ réfléchi présente la forme d'un halo diffus autour de point $\mathbf{r_{in}}$, ce qui traduit que l'hypothèse faite sur l'indice optique n'est pas nécessairement optimale, ce qui sera décrit plus en détails par la suite.

Néanmoins, la matrice focalisée large bande $\mathbf{R_{rr}}$ donne donc accès à un ensemble d'observables pertinentes qu'on va maintenant exploiter pour caractériser de manière quantitative l'échantillon étudié. Premièrement, une image confocale $\Im(\mathbf{r})$ fenêtrée temporellement peut être construite en considérant les éléments suivants de la matrice $\mathbf{R_{rr}}$:

[Math 6]
$$\Im(\mathbf{r}) = R(\mathbf{r}, \mathbf{r}, t = 2\bar{n}z/c_0)$$

La Fig. 3B présente ainsi l'image confocale 303 de la cornée pour $z = 250$ $\mu$m. Cette dernière correspond aux éléments diagonaux de la sous-matrice 302 après avoir été réarrangés sous une forme bidimensionnelle. L'image confocale $\Im(\mathbf{r})$ est un estimateur de la réflectivité de la cornée dans un plan de cohérence associé aux photons simplement diffusés dont le temps de vol est donné par $t = 2\bar{n}z/c_0$.

La qualité de cet estimateur dépend de la qualité de focalisation dans le milieu. On note $h_{in/out}(\mathbf{r}, \mathbf{r_{in/out}})$ la réponse impulsionnelle entre chaque point $\mathbf{r_{in/out}}$ et chaque point $\mathbf{r}$ dans l'échantillon (voir Fig. 1B). Pour quantifier la qualité de focalisation, une observable pertinente est la fonction d'étalement volumique de l'énergie autour du point de focalisation en réflexion (ou RPSF pour « *reflection point spread function* ») à laquelle on peut accéder en mesurant la distribution de l'énergie rétrodiffusée le long des antidiagonales de la sous-matrice $\mathbf{R_{rr}}(z, t)$ qui considère des plans de focalisation identiques entrée et sortie ($z_{in} = z_{out}$):

[Math 7]
$$RPSF(\mathbf{r_p}, \Delta x, \Delta y, t) = \langle |R(\{x_p - \Delta x/2, y_p - \Delta y/2, z_p\}, \{x_p + \Delta x/2, y_p + \Delta y/2, z_p\}, t)|^2 \rangle$$

où $< ... >$ est une moyenne sur les pairs de points $\mathbf{r_{in}}$ et $\mathbf{r_{out}}$ centrés sur le même point $\mathbf{r_p}$ tel que $\mathbf{r_p} = (\mathbf{r_{in}} + \mathbf{r_{out}})/2$. $\Delta\mathbf{r} = \mathbf{r_{out}} - \mathbf{r_{in}}$ est la position relative entre ces deux points (coordonnées $\Delta x$, $\Delta y$). Ce profil d'intensité est pertinent car il permet de sonder les aberrations indépendamment de la réflectivité locale du milieu. En effet, en considérant les PSFs entrée et sortie comme isoplanétiques localement, on peut redéfinir des PSFs locales $h^{(l)}_{in/out}$ invariantes par translation autour de chaque point $\mathbf{r_p}$, tel que $h_{in/out}(\mathbf{r}, \mathbf{r_{in/out}}) = h^{(l)}_{in}(\mathbf{r} - \mathbf{r_{in/out}}, \mathbf{r_p})$. Sous cette hypothèse et pour un milieu de réflectivité aléatoire, la RPSF donne le produit de convolution entre les fonctions d'étalement incohérentes d'entrée et sortie du dispositif d'imagerie :

[Math 8]
$$RPSF(\mathbf{r_p}, \Delta x, \Delta y, t) \propto \left|h^{(l)}_{in}(\{\Delta x, \Delta y, 0\}, \mathbf{r_p})\right|^2 \otimes_{\Delta x, \Delta y} \left|h_{out}(\{\Delta x, \Delta y, 0\}, \mathbf{r_p})\right|^2$$

Pour un objet spéculaire, la RPSF donne accès au produit de convolution entre les fonctions d'étalement cohérentes d'entrée et de sortie :

[Math 9]

$$RPSF\left(\mathbf{r_p}, \Delta x, \Delta y, t\right) \propto \left| h_{in}^{(l)}(\{\Delta x, \Delta y, 0\}, \mathbf{r_p}) \otimes_{\Delta x, \Delta y} h_{out}^{(l)}(\{\Delta x, \Delta y, 0\}, \mathbf{r_p}) \right|^2$$

Dans ces deux régimes asymptotiques, la RPSF ne sonde pas exactement la même quantité physique mais sa distribution spatiale est révélatrice du niveau local d'aberration au point $\mathbf{r_p}$. La Fig. 3B montre la RPSF 305 obtenue dans la cornée opaque à une profondeur $z_p = 250$ $\mu$m. Celle-ci présente l'aspect suivant : une surintensité associée à des photons diffusés par le plan de cohérence et un fond incohérent associé des évènements de diffusion multiple ayant lieu en amont de ce même plan. La position du maximum de la RPSF et l'extension spatiale de la surintensité observée sur la RPSF nous renseigne sur la qualité de focalisation.

La Figure 3B montre que le maximum de la RPSF (305) n'est pas centré en $\Delta r = 0$. Cela est lié notamment à un problème d'alignement du premier interféromètre. L'intensité confocale ne correspond donc à la position relative $\Delta r = 0$ mais à la position $\Delta \mathbf{r}_{max}$ du maximum d'intensité de la RPSF. Une nouvelle image confocale $\mathfrak{I}'(r)$ corrigée de ces problèmes d'alignement peut ainsi être réalisée :

[Math 10]

$$\mathfrak{I}'(\mathbf{r}) = R(\mathbf{r}, \mathbf{r} + \Delta \mathbf{r}_{max}, t = 2\bar{n}z/c_0).$$

L'image 304 montre la nouvelle image confocale obtenue. La comparaison avec l'image confocale brute $\mathfrak{I}(\mathbf{r})$ 303 montre un premier intérêt de la RPSF pour s'affranchir des problèmes d'alignement du dispositif.

Par ailleurs, si, en dehors de ces problèmes d'alignement, le processus de focalisation numérique était idéal, le support de la RPSF s'étendrait sur une seule cellule de résolution. Comme expliqué ci-dessus, la Fig. 3B montre que ce n'est pas le cas de la cornée opaque étudiée.

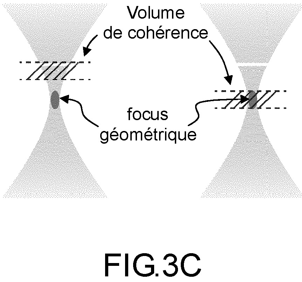

La Fig. 3C illustre la principale raison de cette mauvaise qualité de focalisation : la non-coïncidence des plans de focalisation et de cohérence. Cela peut induire un défaut de mise au point tant à l'émission qu'à la réception du fait de la profondeur de champ finie des objectifs de microscope.

La Fig 4A montre l'intérêt de la RPSF pour ajuster la position des plans de cohérence et de focalisation. Cette figure a été réalisé à partir d'une expérience preuve de concept sur une mire défocalisée d'une distance à $z_m = 100$ $\mu$m sous le plan focal de l'objectif de microscope du bras échantillon (ON=0.3). La Fig 4A montre les RPSF obtenues pour différents plans de focalisation $z = z_{in} = z_{out}$ et pour un plan de cohérence coïncidant avec la position de la mire: $t = 2z_m/c_0$.

Lorsque $z < z_m$ (ou, de manière équivalente, pour $z > z_m$), l'étalement de la RPSF (411, 412) est caractéristique d'un défaut de mise au point. Un estimateur est alors défini afin de déterminer la valeur de $z_m$. Cet estimateur correspond à la position $z_{opt}$ qui maximise l'intensité confocale, i.e le maximum de la RPSF noté $RPSF_{max}$. Pour $z = z_{opt}$, la Fig 4A (421, 422) montre bien que l'extension transverse de RPSF est minimale, i.e. seulement limitée par la diffraction.

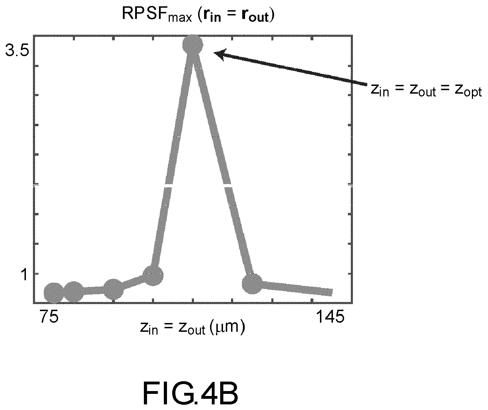

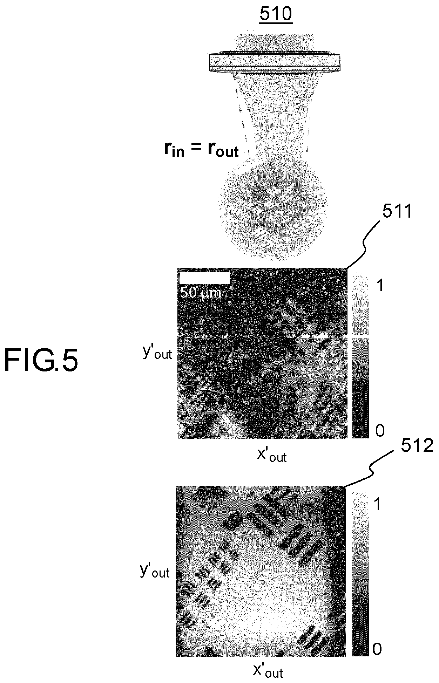

La Fig. 4B montre l'évolution $RPSF_{max}$ en fonction de la profondeur z. On retrouve bien $z_{opt} = z_m$ et l'intensité confocale est 3.5 fois plus intense qu'en dehors de la profondeur de champ ($|z - z_m| > 20$ $\mu$m). La Figure 3C résume le processus d'optimisation de focalisation numérique ainsi réalisé. En maximisant l'intensité confocale ($RPSF_{max}$), on peut faire coïncider le plan de cohérence, correspondant à la nature interférométrique de la mesure, au focus géométrique lié à la position de l'échantillon par rapport à l'objectif de microscope. La Fig. 5 montre l'effet de la double focalisation numérique sur une image confocale de la mire (510). Alors que l'image initiale 511 est dégradée par le défaut de mise au point, le processus de focalisation numérique en entrée et sortie donne une image de la mire 512 avec un contraste optimal et une résolution confocale seulement limitée par la diffraction ($\delta\rho \sim \lambda/(4NA)$). Après correction, tous les détails de la mire sont nets et contrastés. On observe ainsi un effet technique du procédé décrit ci-dessus pour réaliser une image confocale à partir d'un dispositif d'OCT spectral plein champ. Ainsi, il est possible de déterminer, à partir de la première matrice de réflexion volumique focalisée, une cartographie de la réflectivité de l'échantillon.

Un procédé d'optimisation de focalisation similaire est appliqué sur la matrice de réflexion mesurée dans la cornée. Néanmoins, dans l'expérience considérée, la cornée est plongée dans l'eau d'indice connu $n_{eau}$. Surtout, la cornée présente un indice optique $n(\mathbf{r})$ inhomogène spatialement. Nous supposons dans un premier temps que l'indice moyen du milieu est invariant par translation latéralement et que son indice optique $n$ ne dépend que de la profondeur z : $n(\mathbf{r}) = n(z)$. La première étape du procédé est d'amener le plan de focalisation, initialement situé à la profondeur $z_f$ en l'absence d'échantillon, à la surface de l'échantillon situé à la profondeur $z_0$ en appliquant le propagateur $F(., z_0 - z_f, \omega)$ pour un indice $n = n_{eau}$ à la matrice $\tilde{\mathbf{R}}_{\mathbf{uu}}$ :

[Math 11]

$$\tilde{R}'(\mathbf{u}'_{\mathbf{out}}, z_0, \mathbf{u}_{\mathbf{in}}, z_0, \omega) = F(\mathbf{u}'_{\mathbf{out}} - \mathbf{u}_{\mathbf{in}}, z_0 - z_f, \omega)\tilde{R}(\mathbf{u}'_{\mathbf{out}}, \mathbf{u}_{\mathbf{in}}, \omega)F(\mathbf{u}_{\mathbf{in}}, z_0 - z_f, \omega)$$

Un estimateur $\hat{n}(z)$ du profil de l'indice optique $n(z)$ est ensuite obtenu de la manière suivante : pour chaque temps de vol t, on scanne la RPSF en modifiant l'indice optique moyen $\overline{n}$, et par conséquent la position du plan de cohérence $z(\overline{n})$ = $c_0 t/(2\overline{n})$, dans le propagateur $F$ ([Math 5]) de sorte à maximiser l'intensité confocale (RPSF$_{max}$). La valeur de $n$ maximisant cette quantité donne une estimation de l'indice optique intégré $n_{int}(z)$ à la profondeur $z = c_0 t/(2n_{int})$. Le profil de l'indice intégré $n_{int}(z)$ est défini comme l'intégrale de la surface du milieu ($z = z_0$) à la profondeur $z$ de l'indice optique $n(z)$ du milieu :

[Math 12]

$$n_{int}(z) = \frac{1}{z - z_0} \int_{z0}^{z} n(z)dz$$

En discrétisant cette équation par rapport au temps de vol $t$, il est ensuite possible de déduire de l'estimateur $\hat{n}_{int}(z)$ du profil d'indice intégré $n_{int}(z)$ un estimateur $\hat{n}(z)$ de l'indice optique local $n(z)$ au moyen d'une inversion d'un système d'équations linéaires.

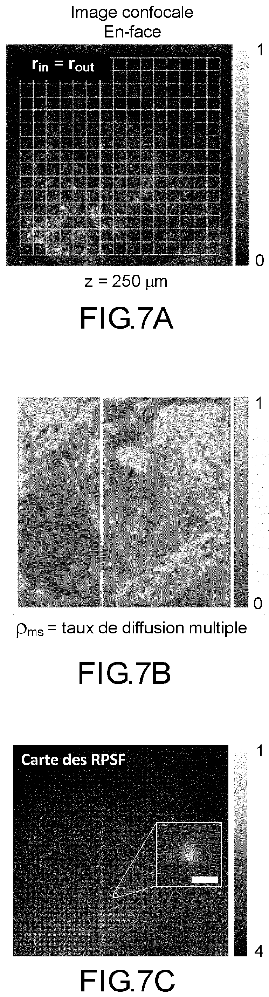

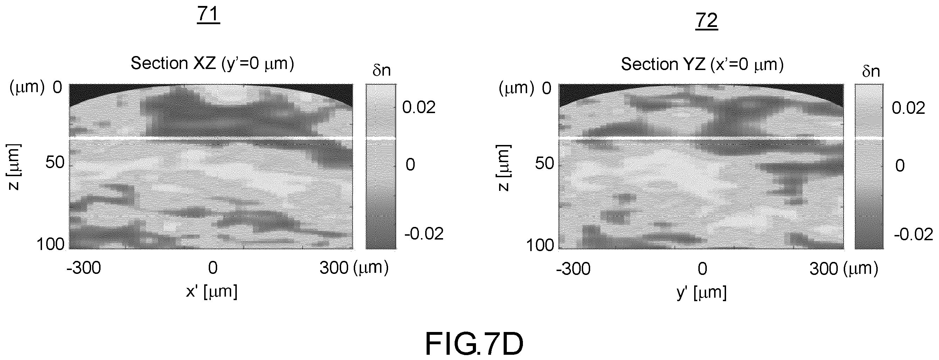

Afin d'obtenir la dépendance de $n$ avec les coordonnées transverses $(x, y)$, une segmentation du champ de vision (cf Fig. 7A) peut être réalisée au préalable sur la matrice de réflexion $\tilde{\mathbf{R}}_{\mathbf{rr}} = [R(\mathbf{r}_{\mathbf{out}}, \mathbf{r}_{\mathbf{in}}, \omega)]$ et le processus décrit plus haut peut être répété pour chaque sous-zone du champ de vision. Un estimateur $\hat{n}(x', y', z)$ de la distribution spatiale de l'indice optique $n(x', y', z)$ dans le milieu peut ainsi être obtenu en considérant des sous zones du champ de vision centrées sur chaque coordonnée transverse $(x', y')$. Deux sections longitudinales (B-scans) de l'indice optique $\hat{n}(x', y', z)$ estimé dans la cornée sont présentées sur la Fig. 7D (images 71, 72). La quantité représentée, $\delta n(x', y', z) = \hat{n}(x', y', z) - \overline{n}$, est l'écart à l'indice optique moyen de la cornée ici estimé à $\overline{n} = 1.4$.

A noter que l'estimateur $\hat{n}$ de la distribution spatiale de l'indice optique $n$ (appelé plus simplement « *cartographie de l'indice optique* » dans la présente description) peut être exploité pour construire un nouveau propagateur $F$ afin de décrire de manière optimale la propagation de la lumière depuis les plans pupilles entrée et sortie du dispositif à l'ensemble des voxels du milieu étudié (i.e la base focalisée). Ce propagateur peut ensuite être utilisé pour construire une nouvelle matrice de réflexion focalisée et fournir une image confocale beaucoup plus proche de la réflectivité réelle du milieu.

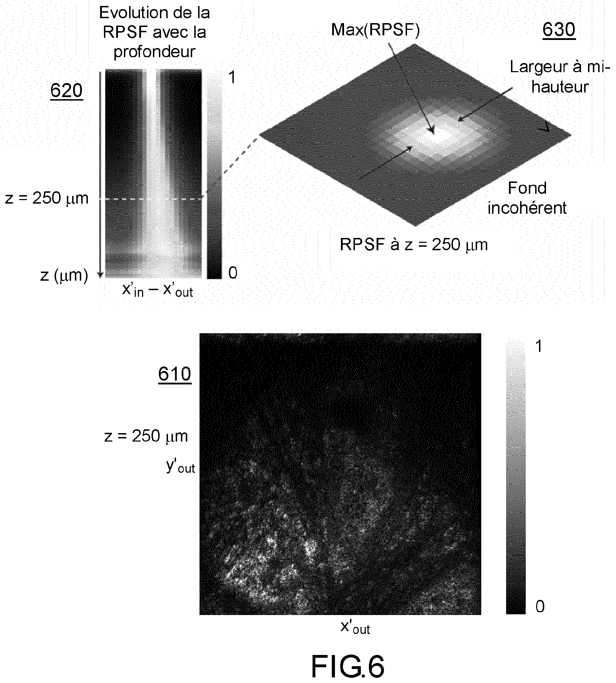

La Fig. 6 montre l'image confocale 610 obtenue à la suite de ce processus de focalisation numérique des données. Notons que l'image peut ainsi être construite en fonction de la profondeur $z$ plutôt qu'en fonction du temps de vol $t$, ce qui est une limite des images OCT conventionnelles dont la dimension axiale est dictée par la différence de marche entre les bras de l'interféromètre.

La comparaison avec l'image confocale précédente $\tilde{\mathfrak{I}}'(\mathbf{r})$ (304, Fig. 3B) illustre le gain obtenu en termes de qualité d'image que ce soit au niveau du contraste et de résolution. Toutefois, la qualité de cette image reste perfectible du fait des phénomènes d'aberrations (hors défaut de mise au point) et de diffusion multiple.

La Fig. 6 illustre cette dernière assertion en montrant une étude quantitative de la RPSF mesurée dans la cornée après focalisation. Elle montre notamment l'évolution en profondeur 620 de la distribution radiale de la RPSF autour du point confocal $\Delta r_{max}$. L'étendue du pic confocal croît avec la profondeur, ce qui quantifie la perte de résolution liée aux aberrations subies par l'onde lumineuse au fur et à mesure de sa propagation dans l'échantillon. A titre d'exemple, l'extension de la RPSF à mi-hauteur 630 est de 15 $\mu$m à une profondeur $z=250$ $\mu$m, ce qui est largement supérieur à la limite de diffraction ($\delta p \sim 1.4$ $\mu$m). Enfin, l'évolution en profondeur de la RPSF montre l'émergence d'un fond incohérent de plus en plus important au-delà de $z = 200$ $\mu$m. Ce dernier résiste à une moyenne sur le temps d'intégration de la caméra, cette composante est déterministe et est donc liée à l'occurrence d'évènements de diffusion multiple en amont du plan focal.

Au-delà de leur évolution en profondeur, les niveaux d'aberration et de diffusion multiple peuvent être également sondés transversalement en étudiant les RPSF mesurées localement autour d'un ensemble de point $\mathbf{r_p}$ du champ de vision.

La Fig. 7A illustre la subdivision du champ de vision réalisée pour extraire les RPSFs locales montrées sur la Fig. 7C. L'étendue spatiale des RPSFs locales montrent par exemple que la qualité de focalisation est bien meilleure en bas à gauche du champ de vision. L'image confocale montrée sur la Fig. 6 est donc un bien meilleur estimateur de la réflectivité du milieu dans cette partie du champ de vision. L'évolution transverse des RPSFs montrent également le caractère non isoplanétique des aberrations induites par les hétérogénéités d'indice optique distribuées dans l'échantillon. Comme nous le verrons par la suite, une compensation locale des aberrations est avantageuse et l'approche matricielle est un outil adapté pour cela.

Au-delà des aberrations, un taux de diffusion multiple sur diffusion simple peut être mesuré à partir de l'intensité confocale $RPSF_{max}$ et du fond incohérent $RPSF_{bg}$ :

[Math 13]

$$\rho_{MS}(\mathbf{r_p}) = \frac{RPSF_{bg}(\mathbf{r_p})}{RPSF_{max}(\mathbf{r_p}) - 2 \times RPSF_{bg}(\mathbf{r_p})}$$

Le facteur 2 au dénominateur prend en compte le phénomène de rétrodiffusion cohérente qui donne lieu à une amplification confocale de l'intensité multiplement diffusée par ce même facteur 2. La Fig. 7B montre une carte du paramètre $\rho_{MS}$ à la profondeur z=250$\mu$m. Cette carte révèle la présence de stries déjà plus ou moins visibles sur l'image confocale de la Fig. 6. De telles stries dans le stroma de la cornée sont caractéristiques de maladies comme le keratocône. Au-delà de cette analyse qualitative, le ratio $\rho_{MS}$ est une première étape vers une image quantitative et locale des propriétés de diffusion de la lumière dans les échantillons biologiques. Suivant l'information recherchée, d'autres paramètres peuvent être extraits comme le taux de diffusion simple (ou taux de diffusion confocale) :

[Math 14]

$$\rho_S(\mathbf{r_p}) = \frac{RPSF_{max}(\mathbf{r_p})}{RPSF_{max}(\mathbf{r_p}) + RPSF_{bg}(\mathbf{r_p})}$$

La décroissance en z de ce paramètre permet de quantifier localement le libre parcours moyen de diffusion dans le milieu. Plus généralement, la quantification des phénomènes de diffusion simple et multiple permet de mesurer localement des paramètres de transport tel que le libre parcours moyen de diffusion, le libre parcours moyen de transport ou encore le coefficient de diffusion L'approche matricielle en réflexion permet en outre d'étudier la croissance du halo diffus à des temps de vol relativement courts, d'où une résolution spatiale bien meilleure que les techniques standards comme la tomographie optique diffuse.

Au-delà de la quantification des phénomènes d'aberration et de diffusion multiple dans le milieu, nous allons maintenant montrer comment, à partir de la matrice de réflexion refocalisée numériquement, il est possible d'effectuer une compensation locale des aberrations induites par l'échantillon afin d'obtenir une image confocale de contraste optimal avec une résolution proche de la limite de diffraction. Pour cela, les matrices de transmission entrée/sortie $\mathbf{T}_{in/out}$ entre une base de correction entrée/sortie et les voxels du milieu peut être estimée. A cette fin, nous faisons ici une analyse locale afin d'estimer des lois de focalisation adaptées pour chaque voxel de l'image.

A titre d'illustration, nous choisissons la base des ondes planes comme base de correction. La première étape du processus de correction matricielle des aberrations consiste à projeter la matrice de réflexion focalisée fenêtrée temporellement, $\mathbf{R_{rr}}(t = 2nz/c_0)$, en sortie (ou entrée) dans cette base de correction par une simple transformée de Fourier spatiale :

[Math 15]

$$\bar{R}(\mathbf{k}_{out}, \mathbf{r_{in}}) = \sum_{\boldsymbol{\rho}_{out}} R(\{\boldsymbol{\rho}'_{out}, \mathbf{z}\}, \{\boldsymbol{\rho}'_{in}, z\}, t = 2nz/c_0)e^{-i\mathbf{k}_{out}\cdot\boldsymbol{\rho}'_{out}}$$

A partir de cette matrice duale $\bar{\mathbf{R}}_{\mathbf{kr}}$, nous construisons une matrice distorsion $\mathbf{D_{kr}}$ définie de la manière suivante :

[Math 16]

$$D(\mathbf{k}_{out}, \mathbf{r_{in}}) = \bar{R}(\mathbf{k}_{out}, \mathbf{r_{in}}) \times T_0(\mathbf{k}_{out}, \mathbf{r_{in}})$$

avec $\mathbf{T_0}$ une matrice de référence qui modélise la propagation des ondes planes si le milieu était homogène (i.e sans aberrations) : $T_0(\mathbf{k}_{out}, \mathbf{r_{in}}) = e^{-i\mathbf{k}_{out}\cdot\mathbf{r_{in}}}$. Dans la Réf [4], il était montré comment, sous une hypothèse isoplanétique, une décomposition en valeurs singulières de la matrice $\mathbf{D}$ permettait d'estimer la transmittance $A(\mathbf{k}_{out})$ de l'aberrateur à partir de la phase de son premier vecteur propre $\mathbf{U_1}$. Ici, puisque les aberrations ne sont pas invariantes spatialement, une subdivision du champ de vision est avantageuse afin d'analyser localement la matrice distorsion et estimer localement des lois de focalisation adaptées pour chaque voxel de l'image. Le champ de vision est divisé en régions se chevauchant définies par leur point central médian $\mathbf{r_p}$ et leur extension spatiale $\Delta\mathbf{r}$. Toutes les composantes distordues du champ associées aux points de focalisation $\mathbf{r_{in}}$ situés dans chaque région peuvent être extraites et stockées dans une matrice distorsion locale :

[Math 17]

$$D'(\mathbf{k}_{out}, \mathbf{r_{in}}, \mathbf{r_p}) = D(\mathbf{k}_{out}, \mathbf{r_{in}}) W_{\Delta\mathbf{r}}(\mathbf{r_{in}} - \mathbf{r_p})$$

où $W_{\Delta\mathbf{r}}(\mathbf{r}) = 1$ pour $|x'| < \Delta x$, $|y'| < \Delta y$ et $|z'| < \Delta z$, et zéro sinon. Idéalement, chaque matrice distorsion locale devrait contenir un ensemble de points de focalisation $\mathbf{r_{in}}$ appartenant à la même tache isoplanétique. En réalité, la condition d'isoplanéité n'est jamais complètement remplie. Un compromis délicat doit donc être fait sur la taille $\Delta\mathbf{r}$ de la fenêtre spatiale $W_{\Delta\mathbf{r}}$ : elle doit être suffisamment petite pour se rapprocher de la condition d'isoplanéité, mais suffisamment grande pour englober un nombre suffisant de réalisations indépendantes du désordre. Une SVD de chaque matrice distorsion locale $D'(\mathbf{r_p})$ donne un premier vecteur propre de sortie $\mathbf{U_1}(\mathbf{r_p}) = [U_1(\mathbf{k}_{out}, \mathbf{r_p})]$. Un estimateur $\hat{\mathbf{T}}_{out}$ de la matrice de transmission $\mathbf{T}_{out} = [T_{out}(\mathbf{k}_{out}, \mathbf{r_p})]$ est ainsi construit :

[Math 18]

$$\hat{T}_{out}(\mathbf{k}_{out}, \mathbf{r_p}) = T_0(\mathbf{k}_{out}, \mathbf{r_p}) \times e^{j\mathrm{arg}\{U_1(\mathbf{k}_{out}, \mathbf{r_p})\}}$$

Un autre estimateur de la matrice de transmission $\mathbf{T}_{out}$ peut être obtenu par un processus de retournement de phase itératif. Il s'appuie sur la relation de récurrence suivante :

[Math 20]

$$\mathbf{W}_{n+1}^{T}(\mathbf{r_p}) = \exp\left\{j\mathrm{arg}\{\mathbf{D}'(\mathbf{r_p})\mathbf{D}'^{\dagger}(\mathbf{r_p})\mathbf{W}_n(\mathbf{r_p})\}\right\}$$

où l'exposant $^T$ désigne l'opération de transposition matricielle et $\mathbf{W}_0(\mathbf{r_p})$ correspond à la transmittance initiale choisie de manière arbitraire par l'opérateur. Celle-ci peut être prise comme homogène, $\mathbf{W}_0(\mathbf{r_p}) = [1 \cdots 1]^T$, ou déterminée à partir de notre connaissance préalable des aberrations dans le milieu afin d'accélérer la convergence du processus itératif. Suivant le choix de l'opérateur, le processus de retournement de phase itératif peut être itéré $m$ fois et le vecteur résultant, $\mathbf{W}_m = [W_m(\mathbf{k}_{out}, \mathbf{r_p})]$, constitue un estimateur de la transmittance de l'aberrateur au point $\mathbf{r_p}$. Un estimateur $\hat{\mathbf{T}}_{out}$ de la matrice de transmission $\hat{\mathbf{T}}_{out} = [T_{out}(\mathbf{k}_{out}, \mathbf{r_p})]$ est ainsi construit :

[Math 21]

$$\hat{T}_{out}(\mathbf{k}_{out}, \mathbf{r_p}) = T_0(\mathbf{k}_{out}, \mathbf{r_p}) \times W_m(\mathbf{r_p})$$

Quel que soit la manière d'estimer la matrice de transmission $\mathbf{T}_{out}$, le conjugué en phase de l'estimateur $\hat{\mathbf{T}}_{out}$ est ensuite appliqué pour corriger les aberrations en sortie du milieu et construire une nouvelle matrice de réflexion focalisée $\overline{\mathbf{R}}_{rr} = [\overline{R}(\rho_{out}, \rho_{in}, z)]$ via le produit matriciel suivant :

[Math 22]

$$\overline{\mathbf{R}}_{rr}(z) = \hat{\mathbf{T}}_{out}^{\dagger} \times \overline{\mathbf{R}}_{kr}(z)$$

où le symbole $\dagger$ correspond à une trans-conjugaison de la matrice $\hat{\mathbf{T}}_{out}$. Le même procédé peut être répété en entrée afin d'estimer la matrice de transmission $\mathbf{T}_{in}$ et itéré en diminuant graduellement l'extension $\Delta r$ des fenêtres spatiales afin de corriger des aberrations d'ordre de plus en plus élevées associées à des patchs d'isoplanétisme de plus en plus petits. A la fin du processus de correction matricielle des aberrations, une image confocale corrigée est obtenue à partir des éléments diagonaux des matrices $\overline{\mathbf{R}}_{rr}(z)$ :

[Math 23]

$$\mathfrak{I}_c(\mathbf{r}) = \bar{R}(\mathbf{r}, \mathbf{r})$$

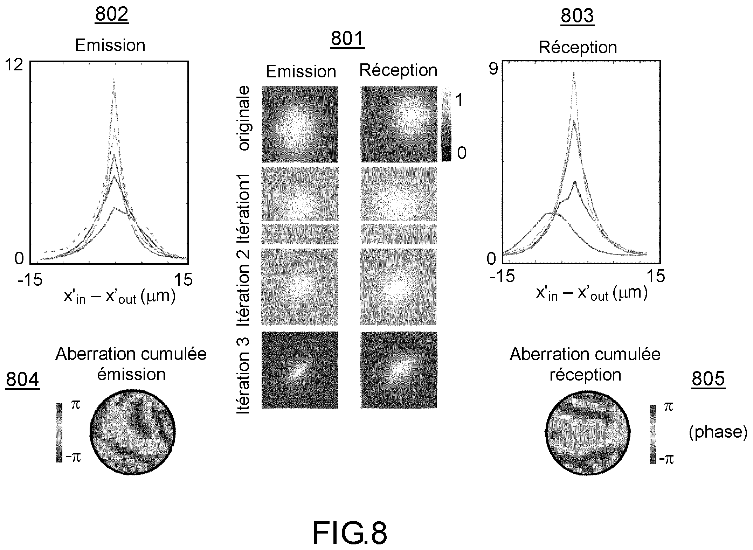

La Fig. 8 montre le résultat de procédé de correction numérique des aberrations transverses dans la cornée à la profondeur z=250 μm. Une méthode de retournement de phase itératif est ici considérée pour estimer les matrices de transmission $\hat{T}_{out}$ et $T_{in}$.

La Fig. 8 montre l'évolution 801, 802, 803 de la RPSF à chaque itération pour une sous-zone du champ de vision de $105 \times 105$ μm². Au fur et à mesure des itérations en entrée et sortie, la RPSF s'affine puisque des aberrations de plus en plus locales sont corrigées. Les lois d'aberration 804, 805 obtenues en entrée et sortie à la fin du processus sont également montrées sur la Fig. 8. Dans un cas idéal, du fait de la réciprocité spatiale, elles devraient être identiques ($T_{in} = T_{out}$). Elles diffèrent notamment du fait des imperfections du système d'imagerie, notamment ses défauts d'alignement. Le processus de correction matricielle des aberrations ne corrige donc pas seulement les aberrations induites par les hétérogénéités d'indice optique dans le milieu mais également les imperfections du système d'imagerie. La comparaison entre les images confocales de la sous-zone avant et après correction, $\mathfrak{I}'(\mathbf{r})$ et $\mathfrak{I}_c(\mathbf{r})$, illustrent l'intérêt d'une correction transverse des aberrations, après une correction axiale de ces dernières.

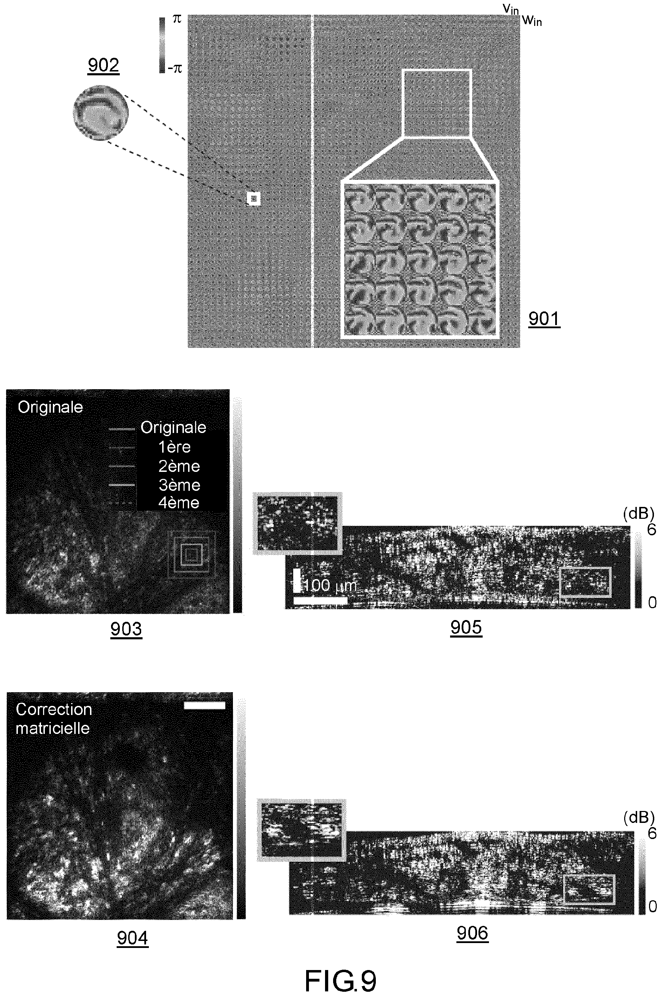

La Fig. 9 montre la phase de la matrice de transmission $T_{in}$ obtenue à la fin du processus itératif de correction des aberrations (901, 902). Celle-ci contient l'ensemble des lois d'aberrations pour chaque sous zone du champ de vision (Fig. 7A). Les images 903, 904 montrent une coupe *en face* de l'image confocale volumique de la cornée à la profondeur $z = 250$ μm avant et après correction numérique des aberrations. Les images 905, 906 montrent une section longitudinale (B-scan) de l'image confocale volumique de la cornée avant et après correction numérique des aberrations. L'image confocale apparaît bien plus contrastée après la correction matricielle des aberrations : l'intensité confocale est augmentée d'un facteur 7 en moyenne sur le champ de vision. Le gain en résolution est également notable : l'étude des RPSFs montre un affinement de la résolution d'un facteur 3.

Les preuves de validations expérimentales ont été réalisées avec un système de caractérisation du type de celui illustré sur la Fig. 1A. Bien entendu, d'autres systèmes de caractérisation conformes à la présente description sont possibles pour la mise en œuvre de procédés selon la présente description.

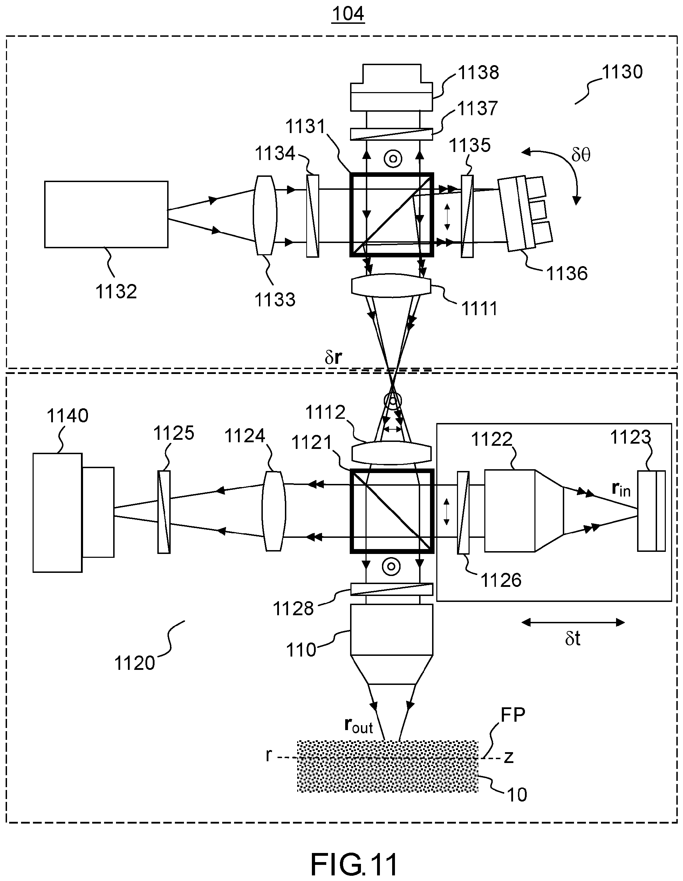

Ainsi, les Fig 10A, Fig. 10B et Fig. 11 représentent des systèmes pour la caractérisation optique d'un échantillon formé d'un milieu volumique et diffusant, selon d'autres exemples de réalisation.

Dans l'exemple du système 102 illustré sur la Fig. 10A, une différence avec le système illustré sur la Fig. 1A réside dans le champ issu du bras de référence. L'onde de référence dans le premier interféromètre provient directement de la source par l'intermédiaire du séparateur de faisceaux 1032. Pour une source lumineuse 132 fibrée (cas considéré dans la Fig. 10A), le bras de référence comprend une fibre optique 1033 et un collimateur 1034 permettant de projeter une onde de référence sous la forme d'une onde plane au niveau du détecteur 140. En espace libre, le bras de référence serait constitué d'un système de lentilles choisi idéalement dans le but de minimiser la différence de marche entre l'onde de référence et les ondes diffusées simplement par les réflecteurs situés dans un plan d'intérêt dans le bras échantillon. Dans ce cas, on mesure l'interférence entre l'onde réfléchie par l'échantillon illuminée par un ensemble de fronts d'onde incidents différents prédéterminés et une onde plane de référence qui ne varie plus qu'en fréquence. On accède ainsi directement au champ rétrodiffusé $E_s(\rho_{out}, u_{in}, \omega)$ par l'échantillon. En multipliant numériquement ce champ par un champ de référence $E_r^*(\rho_{out}, u_{in}, \omega)$, on accède à la matrice $\tilde{R}_{\rho u} = [\tilde{R}(\rho_{out}, u_{in}, \omega)]$.

Dans l'exemple du système 103 illustré sur la Fig. 10B, la source considérée est une source large bande. La mesure de la dépendance spectrale est rendue possible grâce à l'utilisation d'un détecteur 160 comprenant un spectromètre. Le spectromètre comprend par exemple un élément dispersif 161 (par exemple un réseau) et un dispositif de détection 162. Chaque élément du dispositif de détection mesure le champ à une coordonnée $\rho_{out}$ du plan ImP et à une fréquence $\omega$. Le dispositif mesure une matrice $\tilde{R}_{\rho u} = [\tilde{R}(\rho_{out}, u_{in}, \omega)]$ en tout point équivalente à celle mesurée par le système de la Fig. 1A.

Un autre exemple de système de caractérisation optique d'un milieu volumique et diffusant selon la présente description est représenté de façon schématique sur la FIG. 11. Le système 104 présenté sur la FIG. 11 présente l'avantage d'être plus rapide, en termes de temps d'acquisition, et amélioré, en termes de rapport signal-à-bruit, par rapport au système présenté sur la FIG. 1A notamment lorsqu'on souhaite obtenir une image confocale de l'échantillon sur un faible nombre de sections transverses de l'échantillon.

Le système 104 comprend un dispositif d'illumination 1130 avec une source lumineuse 1132 spatialement incohérente agencé au foyer d'une lentille 1133, et configuré pour l'émission d'une pluralité d'ondes lumineuses incidentes destinées à l'illumination à travers l'objectif de microscope 110 d'un champ de vision donné de l'échantillon 10. Le système 104 comprend également un détecteur 1140, agencé au foyer d'une lentille 1124 et une unité de traitement (non représentée) recevant notamment les signaux optoélectroniques issus du détecteur 1140. Le système de caractérisation 104 comprend deux interféromètres placés en série. Un premier interféromètre 1120 et un deuxième interféromètre qui fait partie du dispositif d'illumination 1130. Le deuxième interféromètre est par exemple un interféromètre de Michelson en configuration coin d'air permettant de générer à sa sortie deux faisceaux d'illumination inclinés l'un par rapport à l'autre et de polarisations orthogonales. Le premier interféromètre 1120 est par exemple un interféromètre de Linnik avec un séparateur de faisceaux polarisé 1121 ainsi que des lames quart d'onde 1126, 1128 sur chacun des bras. Un système afocal 1111, 1112 permet de conjuguer les plans des miroirs 1136 et 1138 du deuxième interféromètre avec les plans pupilles des objectifs de microscope 1122 et 110 du premier interféromètre.

Le champ incident, spatialement et temporellement incohérent, est polarisé rectilignement à 45 degrés des directions parallèle (e∥) et normale (e⊥) par rapport au plan du dispositif, au moyen d'un polariseur 1134. Les composantes de cette onde polarisée suivant les directions e∥ et e⊥ sont respectivement transmises et réfléchies par un séparateur de faisceaux polarisés 1131. Chaque bras du deuxième interféromètre contient une lame quart d'onde (1135, 1137) et un miroir (1136, 1138). Sur l'un des bras, le miroir (1136) est incliné par rapport à l'axe optique. A leur retour, les deux ondes issues de chaque bras sortent de l'interféromètre sous forme de deux faisceaux inclinés polarisés orthogonalement. Ces deux faisceaux sont toutefois cohérents entre eux puisqu'issus de la même onde incidente.

Dans le premier interféromètre 1120, les deux faisceaux sont à nouveau séparés par le séparateur de faisceaux polarisés 1121. Le faisceau polarisé suivant e∥ est transmis dans le bras de référence. Le faisceau polarisé suivant e⊥ est réfléchi dans le bras objet. La présence de lames quarts d'ondes 1126, 1128 sur chacun des deux bras permet de transmettre les deux faisceaux de manière optimale une fois ces derniers réfléchis par l'échantillon dans le bras objet et le miroir sur le bras de référence. Ces deux faisceaux sont recombinés en sortie de l'interféromètre à l'aide d'un analyseur 1125 polarisé à 45 degrés par rapport à e∥ et e⊥. Ils peuvent ainsi interférer dans le plan focal de la lentille 1124. Le détecteur, par exemple une caméra CCD ou CMOS enregistre le signal d'interférence correspondant. L'inclinaison du miroir 1136 fait que les faisceaux référence et objet sont décalés l'un par rapport à l'autre sur la caméra. On mesure ainsi la réponse impulsionnelle entre des points $\rho_{in}$ et $\rho_{out}$ distincts. Ainsi, le système décrit au moyen de la Fig. 11, permet de mesurer une matrice de réflexion polychromatique $\mathbf{R}_{\rho\rho}$ dans le domaine temporel et dans une base conjuguée au plan focal de l'objectif du microscope :

[Math 24]

$$R(\boldsymbol{\rho_{out}}, \boldsymbol{\rho_{in}}, t) = \int d\tau\, E_s(\boldsymbol{\rho_{out}}, \boldsymbol{\rho_{in}}, \tau) \times E_r^*(\boldsymbol{\rho_{out}}, \boldsymbol{\rho_{in}} - \boldsymbol{\rho_{out}}, \tau - t)$$

Le temps de vol t des photons est contrôlé par la différence de marche entre les bras de référence et échantillon du premier interféromètre 1120. Le temps de vol peut donc être scanné en déplaçant simultanément les éléments 1122, 1123 et 1126 du bras de référence du premier interféromètre. La position relative des points $\rho_{in}$ et $\rho_{out}$ est contrôlé par l'inclinaison du miroir de référence 1136 dans le deuxième interféromètre.

Dans des exemples de réalisation, en effectuant des transformées de Fourier à la fois temporelle sur la variable t et spatiale sur la coordonnée $\rho_{out}$ sur les coefficients de la matrice $\mathbf{R}_{\rho\rho}$, on obtient une matrice $\tilde{\mathbf{R}}_{u\rho} = [\tilde{R}(\mathbf{u_{out}}, \boldsymbol{\rho_{in}}, \omega)]$, équivalente à celle enregistrée par le dispositif de la Fig 1A. Seules les coordonnées d'entrée et sortie sont échangées. Le même traitement numérique que celui précédemment décrit peut être appliqué simplement en échangeant les coordonnées de sortie et d'entrée de la matrice $\tilde{\mathbf{R}}_{\rho u}$.

Dans d'autres exemples de réalisation, on peut obtenir la matrice de réflexion focalisée $\mathbf{R}_{rr}$ à partir de la matrice $\mathbf{R}_{\rho\rho}$ en appliquant des décalages temporels aux signaux d'interférences mesurés :

[Math 25]

$$R(\mathbf{r_{out}}, \mathbf{r_{in}}, t) = \sum_{\boldsymbol{\rho_{out}}} \sum_{\boldsymbol{\rho_{in}}} A_{in}(\boldsymbol{\rho_{in}}, \mathbf{r_{in}}) A_{out}(\boldsymbol{\rho_{out}}, \mathbf{r_{out}}) R(\boldsymbol{\rho_{out}}, \boldsymbol{\rho_{in}}, t - \Delta t(\boldsymbol{\rho_{in}}, \mathbf{r_{in}}) - \Delta t(\boldsymbol{\rho_{out}}, \mathbf{r_{out}}))$$

où $A_{in/out}$ est un terme d'apodisation spatiale du champ (liés à la décroissance géométrique des fonctions de Green dans la théorie intégrale de la diffraction) et $\Delta t_{in/out}$ est le temps de vol associé au photon balistique se propageant du point $\mathbf{r_{in/out}}$ au plan conjugué au plan d'émission($\rho_{in}$)/détection($\rho_{out}$) dans l'échantillon. Cette opération de décalage temporel est équivalente au propagateur de Fresnel appliqué dans le domaine de Fourier. Si les données sont acquises temporellement, une rétropropagation du champ mesuré dans le domaine temporel peut s'avérer plus avantageuse à la fois en termes de temps et de mémoire par rapport à un passage dans le domaine fréquentiel.

Bien que décrite à travers un certain nombre d'exemples de réalisation détaillés, les procédés et systèmes de caractérisation optique comprennent différentes variantes, modifications et perfectionnements qui apparaîtront de façon évidente à l'homme de l'art, étant entendu que ces différentes variantes, modifications et perfectionnements font partie de la portée de l'invention, telle que définie par les revendications qui suivent.

**Références**

Réf 1 : S. M. Popoff et al. « Measuring the Transmission Matrix in Optics », Phys. Rev. Lett. 104, 100601, 2010.
Réf 2 : A. Badon et al. « Smart optical coherence tomography for ultra-deep imaging through highly scattering media », Sci. Adv. 2016; 2:e1600370.
Réf.3 : US20040061867
Réf. 4 : US20210310787

**Revendications**

1. Procédé de caractérisation optique d'un échantillon (10) formé d'un milieu volumique et diffusant, le procédé comprenant :

   - une étape de positionnement dudit échantillon (10) dans un champ de vision d'un premier objectif de microscope (110), ledit objectif de microscope étant situé dans un bras objet d'un premier interféromètre (120), ledit premier interféromètre comprenant en outre un bras de référence;
   - une étape de génération, au moyen d'un dispositif d'illumination (130) comprenant une source lumineuse (132) à large bande spectrale, d'une première pluralité de $N_{in}$ ondes lumineuses incidentes présentant des fronts d'onde différents ;
   - pour chaque onde lumineuse incidente de front d'onde donné, une étape d'acquisition au moyen d'un détecteur (140) comprenant $N_{out}$ détecteurs élémentaires, d'une deuxième pluralité ($N_\omega$) de signaux d'interférence, chaque signal d'interférence résultant de l'interférence, sur un plan de détection du détecteur, entre une onde rétrodiffusée par l'échantillon illuminé par ladite onde lumineuse incidente, et une onde de référence issue du bras de référence, lesdits signaux d'interférence étant acquis pour $N_\omega$ fréquences différentes ou $N_\omega$ différences de marche différentes entre l'onde rétrodiffusée et l'onde de référence;
   - la détermination d'une matrice réflexion polychromatique tridimensionnelle $\tilde{\mathbf{R}}_{\rho u} = [\tilde{R}(\rho_{out}, \mathbf{u_{in}}, \omega)]$ de taille $N_{in}$ x $N_{out}$ x $N_\omega$, ladite matrice réflexion polychromatique tridimensionnelle comprenant l'ensemble des signaux d'interférence ($N_{in}$ x $N_\omega$) acquis pour les $N_{in}$ ondes lumineuses incidentes et $N_\omega$ fréquences;
   - la détermination numérique, par application d'un premier propagateur, à partir de ladite matrice réflexion polychromatique, d'au moins une première matrice de réflexion volumique focalisée ( $\mathbf{R_{rr}}$), comprenant un ensemble de réponses de l'échantillon entre des points sources et des points de réception conjugués avec des voxels $\mathbf{r_{in}}(x'_{in}, y'_{in}, z_{in})$ et $\mathbf{r_{out}}(x'_{out}, y'_{out}, z_{out})$ de l'échantillon (10) situés respectivement à des profondeurs $z_{in}$ et $z_{out}$ dans l'échantillon;
   - la détermination, à partir de ladite première matrice de réflexion volumique focalisée, d'au moins une cartographie d'un paramètre physique dudit échantillon.

2. Procédé de caractérisation optique selon la revendication 1, dans lequel ladite au moins une cartographie d'un paramètre physique dudit milieu hétérogène comprend :
   une image confocale, une cartographie de la fonction d'étalement de l'énergie autour d'une pluralité de points de focalisation ($\mathbf{r_p}$) en réflexion (RPSF), une cartographie d'un taux de diffusion simple, une cartographie d'un taux de diffusion multiple, une cartographie de l'indice optique de l'échantillon.

3. Procédé de caractérisation optique selon l'une quelconque des revendications précédentes, comprenant en outre :

   - la détermination à partir de ladite matrice de réflexion volumique focalisée d'une première image confocale en réflexion ( $\mathfrak{I}(\mathbf{r})$) ;
   - la détermination, à partir de ladite matrice de réflexion volumique focalisée, d'une cartographie de la fonction d'étalement et la détermination d'une cartographie de la position du maximum d'intensité de chaque fonction d'étalement;
   - la détermination à partir de la première image confocale en réflexion et de ladite cartographie de la position du maximum d'intensité, d'une deuxième image confocale en réflexion ( $\mathfrak{I}'(\mathbf{r})$) corrigée d'un défaut d'alignement et/ou de mise au point du premier interféromètre.

4. Procédé de caractérisation optique selon l'une quelconque des revendications précédentes, comprenant en outre :

- à partir de ladite première matrice de réflexion volumique focalisée, la détermination d'une pluralité de cartographies de la fonction d'étalement pour une pluralité de valeurs d'un indice optique intégré de l'échantillon ;
- à partir de ladite pluralité de cartographies de la fonction d'étalement, la détermination d'une pluralité de cartographies de la position du maximum d'intensité de la fonction d'étalement, chaque cartographie étant obtenue pour une valeur de l'indice optique intégré ;
- la détermination d'une cartographie de la valeur optimale de l'indice optique intégré pour laquelle, en chaque point de focalisation, la valeur maximale d'intensité de la fonction d'étalement est la plus grande;
- la détermination numérique d'un deuxième propagateur basé sur ladite cartographie de la valeur optimale de l'indice optique intégré; et

la détermination d'une deuxième matrice de réflexion volumique focalisée utilisant ledit deuxième propagateur.

5. Procédé selon la revendication 4, comprenant en outre, à partir de ladite cartographie de la valeur optimale de l'indice optique intégré, la détermination d'une cartographie de l'indice optique du milieu en chaque point de focalisation.

6. Procédé de caractérisation optique selon la revendication 5, dans lequel ledit deuxième propagateur est déterminé sur la base de ladite cartographie de l'indice optique.

7. Procédé selon l'une quelconque des revendications 4 à 6, comprenant en outre :

- la détermination, à partir de la deuxième matrice de réflexion volumique focalisée, d'une matrice distorsion (**D**) définie entre une base de correction et une base focalisée, comprenant :
- la projection, en entrée ou en sortie, de la deuxième matrice de réflexion volumique focalisée, dans la base de correction, pour obtenir une matrice de réflexion projetée($\overline{\mathbf{R}}_{\mathbf{kr}}$) respectivement en entrée ou en sortie;
- une détermination de la matrice distorsion par produit terme à terme entre ladite matrice de réflexion projetée et une matrice de réflexion de référence, définie pour un milieu de référence, dans ladite base de correction;
- une détermination locale des invariants de ladite matrice distorsion, afin d'identifier, dans la base de correction, des lois d'aberration dans des sous domaines du champ de vision ;
- l'estimation à partir desdites lois d'aberration, d'une matrice de transmission ($\mathbf{T}_{\mathbf{out}}$) entre des voxels du champ de vision et la base de correction.

8. Procédé de caractérisation optique selon l'une quelconque des revendications précédentes, dans lequel :

- ladite source lumineuse est une source à longueur d'onde variable et pour chaque onde lumineuse incidente de front d'onde prédéterminé, les signaux d'interférence de ladite deuxième pluralité ($N_\omega$) de signaux d'interférence sons acquis pour $N_\omega$ longueurs d'onde centrales de ladite onde lumineuse incidente et une différence de marche fixe entre le bras objet et le bras de référence du premier interféromètre.

9. Procédé de caractérisation optique selon l'une quelconque des revendications 1 à 7, dans lequel :

- pour chaque onde lumineuse incidente de front d'onde donné, les signaux d'interférence de ladite deuxième pluralité ($N_\omega$) de signaux d'interférence sont acquis pour $N_\omega$ différences de marche entre le bras objet et le bras de référence du premier interféromètre.

10. Procédé de caractérisation optique selon l'une quelconque des revendications précédentes, dans lequel les $N_{in}$ ondes lumineuses incidentes sont cohérentes spatialement et présentent des fronts d'onde contrôlés par des moyens de mise en forme spatiale du front d'onde.

11. Procédé de caractérisation optique selon la revendication 10, dans lequel lesdites $N_{in}$ ondes lumineuses incidentes de la première pluralité d'ondes lumineuses sont des ondes planes présentant chacune un vecteur d'onde avec une direction différente.

12. Procédé de caractérisation optique selon l'une quelconque des revendications 1 à 9, dans lequel ladite source lumineuse est une source à faible cohérence spatiale, le procédé comprenant en outre :

- la génération, à partir de chaque onde lumineuse incohérente spatialement ou partiellement cohérente issue de la source lumineuse, et au moyen d'un deuxième interféromètre, de deux ondes d'illumination polarisées de polarisations orthogonales et présentant un décalage spatial dans un plan conjugué avec un plan focal (FP) du premier objectif de microscope;

- la variation dudit décalage spatial pour générer les $N_{in}$ ondes incidentes de front d'onde différents ;

- l'envoi, pour chaque décalage spatial, desdites ondes polarisées de polarisations orthogonales respectivement sur les bras objet et de référence dudit premier interféromètre au moyen d'un élément séparateur de polarisation (1121) ;

- l'acquisition au moyen du détecteur (1140), de ladite deuxième pluralité ($N_\omega$) de signaux d'interférence, chaque signal d'interférence résultant de l'interférence, sur le plan de détection du détecteur, entre une onde rétrodiffusée par l'échantillon illuminé par l'une desdites ondes polarisées de polarisations orthogonales, et une onde de référence générée par la réflexion de l'autre desdites ondes polarisées de polarisations orthogonales par un miroir de référence du bras de référence, lesdits signaux d'interférence étant acquis pour $N_\omega$ différences de marche différentes entre l'onde rétrodiffusée et l'onde de référence ;

- ladite matrice réflexion polychromatique étant déterminée à partir de l'ensemble des signaux d'interférence ($N_{in}$ x $N_\omega$) acquis pour les $N_{in}$ décalages spatiaux et $N_\omega$ différences de marche.

13. Système de caractérisation optique d'un échantillon (10) formé d'un milieu volumique et diffusant, comprenant :

- un premier interféromètre (120) avec un bras objet et un bras de référence, le bras objet comprenant un premier objectif de microscope (110) avec un champ de vision donné dans lequel est positionné, en opération, l'échantillon (10);

- un dispositif d'illumination (130) comprenant une source lumineuse (132) à large bande spectrale, configuré pour générer une première pluralité ($N_{in}$) d'ondes lumineuses incidentes présentant des fronts d'onde différents ;

- un détecteur (140) comprenant $N_{out}$ détecteurs élémentaires, configuré pour l'acquisition, pour chaque onde lumineuse incidente de front d'onde donné, d'une deuxième pluralité ($N_\omega$) de signaux d'interférence, chaque signal d'interférence résultant de l'interférence, sur un plan de détection du détecteur, entre une onde rétrodiffusée par l'échantillon illuminé par ladite onde lumineuse incidente, et une onde de référence issue du bras de référence, lesdits signaux d'interférence étant acquis pour $N_\omega$ fréquences différentes ou $N_\omega$ différences de marche différentes entre l'onde rétrodiffusée et l'onde de référence;

- une unité de traitement (150) configurée pour :

- la détermination d'une matrice réflexion polychromatique tridimensionnelle $\tilde{\mathbf{R}}_{\rho u} = [\tilde{R}(\rho_{out}, \mathbf{u_{in}}, \omega)]$ de taille $N_{in}$ x $N_{out}$ x $N_\omega$, ladite matrice réflexion polychromatique tridimensionnelle comprenant l'ensemble des signaux d'interférence ($N_{in}$ x $N_\omega$) acquis pour les $N_{in}$ ondes lumineuses incidentes et $N_\omega$ fréquences;

- la détermination numérique, par application d'un premier propagateur, à partir de ladite matrice réflexion polychromatique, d'au moins une première matrice de réflexion volumique focalisée ($\mathbf{R_{rr}}$), comprenant un ensemble de réponses de l'échantillon entre des points sources et des points de réception conjugués avec des voxels $\mathbf{r_{in}}(x'_{in}, y'_{in}, z_{in})$ et $\mathbf{r_{out}}(x'_{out}, y'_{out}, z_{out})$ de l'échantillon (10) situés respectivement à des profondeurs $z_{in}$ et $z_{out}$ dans l'échantillon;

- la détermination, à partir de ladite première matrice de réflexion volumique focalisée, d'au moins une cartographie d'un paramètre physique dudit échantillon.

14. Système de caractérisation optique selon la revendication 13, dans lequel ledit premier interféromètre est un interféromètre de Linnik et le bras de référence comprend un miroir de référence et un deuxième objectif de microscope.

15. Système selon l'une quelconque des revendications 13 à 14, dans lequel :

- la source lumineuse est une source à faible cohérence spatiale; et le dispositif d'illumination (1130) comprend
- un deuxième interféromètre configuré pour générer à partir de chaque onde lumineuse incohérente spatialement ou partiellement cohérente issue de la source lumineuse, deux ondes d'illumination polarisées de polarisations orthogonales et présentant un décalage spatial dans un plan conjugué avec un plan focal (FP) du premier objectif de microscope (110); et
- des moyens de variation du décalage spatial ; et dans lequel
- ledit premier interféromètre (1120) est un interféromètre de Linnik et comprend
- un élément séparateur de polarisation (1121) configuré pour envoyer respectivement sur les bras objet et de référence, chacune desdites ondes polarisées de polarisations orthogonales et présentant ledit décalage spatial ;
- des moyens de variations de la différence de marche entre les bras objet bras de référence ; et dans lequel

- chaque signal d'interférence de ladite deuxième pluralité ($N_\omega$) de signaux d'interférence résulte de l'interférence, sur le plan de détection du détecteur, entre une onde rétrodiffusée par l'échantillon illuminé par l'une desdites ondes polarisées de polarisations orthogonales, et une onde de référence générée par la réflexion de l'autre desdites ondes polarisées de polarisations orthogonales par un miroir de référence du bras de référence, lesdits signaux d'interférence étant acquis pour $N_\omega$ différences de marche différentes entre l'onde rétrodiffusée et l'onde de référence ;

- ladite matrice réflexion polychromatique est déterminée à partir de l'ensemble des signaux d'interférence ($N_{in}$ x $N_\omega$) acquis pour les $N_{in}$ décalages spatiaux et $N_\omega$ différences de marche.

**Patentansprüche**

1. Verfahren zur optischen Charakterisierung einer Probe (10), die aus einem volumetrischen und streuenden Medium gebildet ist, wobei das Verfahren umfasst:

   - einen Schritt des Positionierens der Probe (10) in einem Sichtfeld eines ersten Mikroskopobjektivs (110), wobei sich das Mikroskopobjektiv in einem Objektarm eines ersten Interferometers (120) befindet, wobei das erste Interferometer ferner einen Referenzarm umfasst;
   - einen Schritt des Erzeugens, unter Verwendung einer Beleuchtungsvorrichtung (130), die eine Breitband-Spektrallichtquelle (132) umfasst, einer ersten Vielzahl von $N_{in}$ einfallenden Lichtwellen mit unterschiedlichen Wellenfronten;
   - für jede einfallende Lichtwelle mit einer gegebenen Wellenfront, einen Schritt des Erfassens mittels eines Detektors (140), der $N_{out}$ elementare Detektoren umfasst, einer zweiten Vielzahl ($N_\omega$) von Interferenzsignalen, wobei jedes Interferenzsignal aus der Interferenz auf einer Detektionsebene des Detektors zwischen einer von der mit der einfallenden Lichtwelle beleuchteten Probe rückgestreuten Welle und einer Referenzwelle aus dem Referenzarm resultiert, wobei die Interferenzsignale für $N_\omega$ unterschiedliche Frequenzen oder $N_\omega$ unterschiedliche Weglängenunterschiede zwischen der rückgestreuten Welle und der Referenzwelle erfasst werden;
   - Bestimmen einer dreidimensionalen polychromatischen Reflexionsmatrix $\tilde{\mathbf{R}}_{pu} = [\tilde{R}(\rho_{out}, \mathbf{u}_{in}, \omega)]$ der Größe $N_{in}$ x $N_{out}$ x $N_\omega$), wobei die dreidimensionale polychromatische Reflexionsmatrix die Gesamtheit der Interferenzsignale ($N_{in}$ x $N_\omega$), die für die $N_{in}$ einfallende Lichtwellen und $N_\omega$ Frequenzen erfasst werden, umfasst;
   - numerisches Bestimmen, durch Anwenden eines ersten Propagators, aus der polychromatischen Reflexionsmatrix, mindestens einer ersten fokussierten Volumenreflexionsmatrix ($\mathbf{R}_{rr}$), die eine Gesamtheit von Antworten der Probe zwischen Quellpunkten und Empfangspunkten umfasst, die mit Voxeln $r_{in}(x'_{in}, y'_{in}, z_{in})$ und $\mathbf{r}_{out}(x'_{out}, y'_{out}, z_{out})$ der Probe (10) konjugiert sind, die sich jeweils in Tiefen $z_{in}$ und $z_{out}$ in der Probe befinden;
   - Bestimmen, aus der ersten fokussierten Volumenreflexionsmatrix mindestens einer Kartierung eines physikalischen Parameters der Probe.

2. Optisches Charakterisierungsverfahren nach Anspruch 1, wobei die mindestens eine Kartierung eines physikalischen Parameters des heterogenen Mediums umfasst: ein konfokales Bild, eine Kartierung der Energieverteilungsfunktion um eine Vielzahl von Brennpunkten ($r_P$) in Reflexion (RPSF), eine Kartierung einer einfachen Streurate, eine Kartierung einer Mehrfachstreurate, eine Kartierung eines optischen Index der Probe.

3. Optisches Charakterisierungsverfahren nach einem der vorstehenden Ansprüche, ferner umfassend:

   - Bestimmen eines ersten konfokalen Reflexionsbildes ($\Im(\mathbf{r})$) aus der fokussierten Volumenreflexionsmatrix;
   - Bestimmen, aus der fokussierten Volumenreflexionsmatrix, einer Kartierung der Verteilungsfunktion, und Bestimmen einer Kartierung der Position der maximalen Intensität jeder Verteilungsfunktion;
   - Bestimmen, aus dem ersten konfokalen Reflexionsbild und der Kartierung der Position der maximalen Intensität, eines zweiten konfokalen Reflexionsbildes ($\Im'(\mathbf{r})$), das für einen Ausrichtungs- und/oder Fokussierungsfehler des ersten Interferometers korrigiert wurde.

4. Optisches Charakterisierungsverfahren nach einem der vorstehenden Ansprüche, ferner umfassend:

   - aus der ersten fokussierten Volumenreflexionsmatrix, Bestimmen einer Vielzahl von Kartierungen der Verteilungsfunktion für eine Vielzahl von Werten des integrierten optischen Index der Probe;
   - aus der Vielzahl von Kartierungen der Verteilungsfunktion, Bestimmen einer Vielzahl von Kartierungen der Position der maximalen Intensität der Verteilungsfunktion, wobei jede Kartierung für einen Wert des integrierten optischen Index erhalten wird;
- Bestimmen einer Kartierung des optimalen Wertes des integrierten optischen Index, für den an jedem Brennpunkt der maximale Intensitätswert der Verteilungsfunktion am größten ist;
- numerisches Bestimmen eines zweiten Propagators basierend auf der Kartierung des optimalen Wertes des integrierten optischen Index; und
- Bestimmen einer zweiten fokussierten Volumenreflexionsmatrix unter Verwendung des zweiten Propagators.

5. Verfahren nach Anspruch 4, ferner umfassend, aus der Kartierung des optimalen Wertes des integrierten optischen Index, Bestimmen einer Kartierung des optischen Index des Mediums an jedem Brennpunkt.

6. Optisches Charakterisierungsverfahren nach Anspruch 5, wobei der zweite Propagator basierend auf der Kartierung des optischen Index bestimmt wird.

7. Verfahren nach einem der Ansprüche 4 bis 6, ferner umfassend:

- Bestimmen, aus der zweiten fokussierten Volumenreflexionsmatrix, einer Verzerrungsmatrix ($D$), die zwischen einer Korrekturbasis und einer fokussierten Basis definiert ist, umfassend:
- Projizieren, am Eingang oder Ausgang, der zweiten fokussierten Volumenreflexionsmatrix in die Korrekturbasis, um eine projizierte Reflexionsmatrix ($\overline{R}_{kr}$) jeweils am Eingang oder Ausgang zu erhalten;
- Bestimmen der Verzerrungsmatrix durch ein gliedweises Produkt zwischen der projizierten Reflexionsmatrix und einer für ein Referenzmedium definierten Referenzreflexionsmatrix in der Korrekturbasis;
- lokales Bestimmen der Invarianten der Verzerrungsmatrix, um, in der Korrekturbasis, Aberrationsgesetze in Teilbereichen des Sichtfeldes zu identifizieren;
- Schätzen, aus den Aberrationsgesetzen, einer Übertragungsmatrix ($T_{out}$) zwischen Voxeln im Sichtfeld und der Korrekturbasis.

8. Optisches Charakterisierungsverfahren nach einem der vorstehenden Ansprüche, wobei:

- die Lichtquelle eine Quelle mit variabler Wellenlänge ist, und für jede einfallende Lichtwelle mit einer vorbestimmten Wellenfront die Interferenzsignale der zweiten Vielzahl ($N_{\omega}$) von Interferenzsignalen für $N_{\omega}$ zentrale Wellenlängen der einfallenden Lichtwelle und ein fester Weglängenunterschied zwischen dem Objektarm und dem Referenzarm des ersten Interferometers erfasst werden.

9. Optisches Charakterisierungsverfahren nach einem der Ansprüche 1 bis 7, wobei:

- für jede einfallende Lichtwelle mit einer gegebenen Wellenfront die Interferenzsignale der zweiten Vielzahl ($N_{\omega}$) von Interferenzsignalen für $N_{\omega}$ Weglängenunterschiede zwischen dem Objektarm und dem Referenzarm des ersten Interferometers erfasst werden.

10. Optisches Charakterisierungsverfahren nach einem der vorstehenden Ansprüche, wobei die $N_{in}$ einfallenden Lichtwellen räumlich kohärent sind und Wellenfronten aufweisen, die unter Verwendung von räumlicher Wellenfrontformung gesteuert werden.

11. Optisches Charakterisierungsverfahren nach Anspruch 10, wobei die $N_{in}$ einfallenden Lichtwellen der ersten Vielzahl von Lichtwellen flache Wellen sind, die jeweils einen Wellenvektor mit unterschiedlicher Richtung aufweisen.

12. Optisches Charakterisierungsverfahren nach einem der Ansprüche 1 bis 9, wobei die Lichtquelle eine Quelle mit geringer räumlicher Kohärenz ist, wobei das Verfahren ferner umfasst:

- Erzeugen, aus jeder räumlich inkohärenten oder teilweise kohärenten Lichtwelle der Lichtquelle, und unter Verwendung eines zweiten Interferometers, von zwei polarisierten Beleuchtungswellen mit orthogonalen Polarisationen, die eine räumliche Verschiebung in einer Ebene aufweisen, die mit der Brennebene (FP) des ersten Mikroskopobjektivs konjugiert ist;
- Variieren der räumlichen Verschiebung zum Erzeugen der $N_{in}$ einfallenden Wellen mit unterschiedlichen Wellenfronten;
- Senden, für jede räumliche Verschiebung, der polarisierten Wellen orthogonaler Polarisationen auf den Objekt- bzw. Referenzarm des ersten Interferometers unter Verwendung eines Polarisationstrennelements (1121);

- Erfassen, unter Verwendung des Detektors (1140), der zweiten Vielzahl ($N_\omega$) von Interferenzsignalen, wobei jedes Interferenzsignal aus der Interferenz auf der Detektionsebene des Detektors zwischen einer von der Probe, die von einer der polarisierten Wellen orthogonaler Polarisationen beleuchtet wird, rückgestreuten Welle und einer Referenzwelle resultiert, die durch die Reflexion der anderen der polarisierten Wellen orthogonaler Polarisationen durch einen Referenzspiegel des Referenzarms erzeugt wird, wobei die Interferenzsignale für $N_\omega$ unterschiedliche Weglängenunterschiede zwischen der rückgestreuten Welle und der Referenzwelle erfasst werden;
- die polychromatische Reflexionsmatrix aus der Gesamtheit der Interferenzsignale ($N_{in}$ x $N_\omega$) bestimmt wird, die für die $N_{in}$ räumlichen Verschiebungen und $N_\omega$ Weglängenunterschiede erfasst werden.

**13.** System zur optischen Charakterisierung einer Probe (10), die aus einem volumetrischen und streuenden Medium gebildet ist, umfassend:

- ein erstes Interferometer (120) mit einem Objektarm und einem Referenzarm, wobei der Objektarm ein erstes Mikroskopobjektiv (110) mit einem gegebenen Sichtfeld umfasst, in dem die Probe (10) im Betrieb positioniert ist;
- eine Beleuchtungsvorrichtung (130), die eine Breitband-Spektrallichtquelle (132) umfasst, die konfiguriert ist, um eine erste Vielzahl ($N_{in}$) von einfallenden Lichtwellen mit unterschiedlichen Wellenfronten zu erzeugen;
- einen Detektor (140), der $N_{out}$ elementare Detektoren umfasst, und für die Erfassung, für jede einfallende Lichtwelle einer gegebenen Wellenfront, einer zweiten Vielzahl ($N_\omega$) von Interferenzsignalen konfiguriert ist, wobei jedes Interferenzsignal aus der Interferenz, auf einer Detektionsebene des Detektors, zwischen einer von der mit der einfallenden Lichtwelle beleuchteten Probe rückgestreuten Welle und einer Referenzwelle aus dem Referenzarm resultiert, wobei die Interferenzsignale für $N_\omega$ unterschiedliche Frequenzen oder $N_\omega$ unterschiedliche Weglängenunterschiede zwischen der rückgestreuten Welle und der Referenzwelle erfasst werden;
- eine Verarbeitungseinheit (150), die konfiguriert ist zum:
- Bestimmen einer dreidimensionalen polychromatischen Reflexionsmatrix $\tilde{\mathbf{R}}_{pu} = [\tilde{R}(\rho_{out}, \mathbf{u}_{in}, \omega)]$ der Größe $N_{in}$ x $N_{out}$ x $N_\omega$, wobei die dreidimensionale polychromatische Reflexionsmatrix die Gesamtheit der Interferenzsignale ($N_{in}$ x $N_\omega$), die für die $N_{in}$ einfallenden Lichtwellen und $N_\omega$ Frequenzen erfasst werden, umfasst;
- numerisches Bestimmen, durch Anwenden eines ersten Propagators, aus der polychromatischen Reflexionsmatrix, mindestens einer ersten fokussierten Volumenreflexionsmatrix ($\mathbf{R}_{rr}$), die eine Gesamtheit von Antworten der Probe zwischen Quellpunkten und Empfangspunkten umfasst, die mit Voxeln $r_{in}(x'_{in}, y'_{in}, z_{in})$ und $\mathbf{r}_{out}(x'_{out}, y'_{out}, z_{out})$ der Probe (10) konjugiert sind, die sich jeweils in Tiefen $z_{in}$ und $z_{out}$ in der Probe befinden;
- Bestimmen, aus der ersten fokussierten Volumenreflexionsmatrix mindestens einer Kartierung eines physikalischen Parameters der Probe.

**14.** Optisches Charakterisierungssystem nach Anspruch 13, wobei das erste Interferometer ein Linnik-Interferometer ist und der Referenzarm einen Referenzspiegel und ein zweites Mikroskopobjektiv umfasst.

**15.** System nach einem der Ansprüche 13 bis 14, wobei:

- die Lichtquelle eine Quelle mit geringer räumlicher Kohärenz ist; und die Beleuchtungsvorrichtung (1130) umfasst:
- ein zweites Interferometer, das konfiguriert ist, um aus jeder räumlich inkohärenten oder teilweise kohärenten Lichtwelle, die von der Lichtquelle ausgeht, zwei polarisierte Beleuchtungswellen orthogonaler Polarisationen zu erzeugen, die eine räumliche Verschiebung in einer Ebene aufweisen, die mit der Brennebene (FP) des ersten Mikroskopobjektivs (110) konjugiert ist; und
- Mittel zur Variation der räumlichen Verschiebung; und wobei
- das erste Interferometer (1120) ein Linnik-Interferometer ist und umfasst:
- ein Polarisationstrennelement (1121), das konfiguriert ist, um jeweils auf den Objekt- und Referenzarm polarisierte Wellen orthogonaler Polarisationen und aufweisend die räumliche Verschiebung zu senden;
- Mittel zur Variation des Weglängenunterschieds zwischen dem Objektarm und dem Referenzarm; und wobei
- jedes Interferenzsignal von der zweiten Vielzahl ($N_\omega$) aus Interferenzsignalen durch die Interferenz auf der Detektionsebene des Detektors zwischen einer von der Probe, die von einer der polarisierten Wellen orthogonaler Polarisationen beleuchtet wird, rückgestreuten Welle und einer Referenzwelle, die durch die Reflexion der anderen polarisierten Welle orthogonaler Polarisationen durch einen Referenzspiegel des Referenzarms erzeugt wird, resultiert, wobei die Interferenzsignale für $N_\omega$ unterschiedliche Weglängenunterschiede zwischen der rückgestreuten Welle und der Referenzwelle erfasst werden;
- die polychromatische Reflexionsmatrix aus der Gesamtheit der Interferenzsignale ($N_{in}$ x $N_\omega$) bestimmt wird, die für die $N_{in}$ räumlichen Verschiebungen und $N_\omega$ Weglängenunterschiede erfasst werden.

**Claims**

1. A method for the optical characterization of a sample (10) formed of a bulk scattering medium, the method comprising:

   - a step of positioning said sample (10) in a field of view of a first microscope objective (110), said microscope objective being located in an object arm of a first interferometer (120), said first interferometer further comprising a reference arm;
   - a step of generating, by means of an illuminating device (130) comprising a wide spectral band light source (132), a first plurality of $N_{in}$ incident light waves having different wavefronts;
   - for each incident light wave of given wavefront, a step of acquiring, by means of a detector (140) comprising $N_{out}$ elementary detectors, a second plurality ($N_\omega$) of interference signals, each interference signal resulting from the interference, in a detection plane of the detector, between a wave backscattered by the sample illuminated by said incident light wave and a reference wave from the reference arm, said interference signals being acquired for $N_\omega$ different frequencies or $N_\omega$ different path differences between the backscattered wave and the reference wave;
   - determining a three-dimensional polychromatic reflection matrix $\tilde{R}_{\rho u} = [\tilde{R}(\rho_{out}, u_{in}, \omega)]$ of size $N_{in} \times N_{out} \times N_\omega$, said three-dimensional polychromatic reflection matrix comprising all of the interference signals ($N_{in} \times N_\omega$) acquired for the $N_{in}$ incident light waves and $N_\omega$ frequencies;
   - numerically determining, by applying a first propagator, on the basis of said polychromatic reflection matrix, at least a first focused volumetric reflection matrix ($R_{rr}$), comprising a set of responses for the sample between source points and receiving points that are conjugate with voxels $r_{in}(x'_{in}, y'_{in}, z_{in})$ and $r_{out}(x'_{out}, y'_{out}, z_{out})$ of the sample (10) which are located at depths $z_{in}$ and $z_{out}$ in the sample, respectively;
   - determining, on the basis of said first focused volumetric reflection matrix, at least one map of a physical parameter of said sample.

2. The optical characterization method as claimed in claim 1, wherein said at least one map of a physical parameter of said heterogeneous medium comprises: a confocal image, a map of the point spread function around a plurality of reflection focal points ($r_p$) (RPSF), a map of a single scattering rate, a map of a multiple scattering rate, a map of the optical index of the sample.

3. The optical characterization method as claimed in any one of the preceding claims, the method further comprising:

   - determining, on the basis of said focused volumetric reflection matrix, a first reflection confocal image ($\tilde{\Im}(r)$);
   - determining, on the basis of said focused volumetric reflection matrix, a map of the spread function and determining a map of the position of the intensity maximum of each spread function;
   - determining, on the basis of the first reflection confocal image and said map of the position of the intensity maximum, a second reflection confocal image ($\tilde{\Im}'(r)$) corrected for an alignment and/or focusing defect of the first interferometer.

4. The optical characterization method as claimed in any one of the preceding claims, the method further comprising:

   - on the basis of said first focused volumetric reflection matrix, determining a plurality of maps of the spread function for a plurality of values of an integrated optical index of the sample;
   - on the basis of said plurality of maps of the spread function, determining a plurality of maps of the position of the intensity maximum of the spread function, each map being obtained for a value of the integrated optical index;
   - determining a map of the optimum value of the integrated optical index for which, at each focal point, the maximum intensity value of the spread function is the greatest;
   - numerically determining a second propagator based on said map of the optimum value of the integrated optical index; and

   determining a second focused volumetric reflection matrix using said second propagator.

5. The method as claimed in claim 4, the method further comprising, on the basis of said map of the optimum value of the integrated optical index, determining a map of the optical index of the medium at each focal point.

6. The optical characterization method as claimed in claim 5, wherein said second propagator is determined on the basis of said map of the optical index.

7. The method as claimed in any one of claims 4 to 6, the method further comprising:

- determining, on the basis of the second focused volumetric reflection matrix, a distortion matrix **(D)** defined between a correction base and a focused base, comprising:
- projecting, at entrance or at exit, the second focused volumetric reflection matrix, in the correction base, to obtain a projected reflection matrix ($\overline{\mathbf{R}}_{\mathbf{kr}}$) at entrance or at exit, respectively;
- determining the distortion matrix via term-by-term product between said projected reflection matrix and a reference reflection matrix, defined for a reference medium, in said correction base;
- locally determining the invariants of said distortion matrix, in order to identify, in the correction base, aberration laws in sub-domains of the field of view;
- estimating, on the basis of said aberration laws, a transmission matrix ($\mathbf{T}_{\mathbf{out}}$) between voxels of the field of view and the correction base.

8. The optical characterization method as claimed in any one of the preceding claims, wherein:

- said light source is a variable-wavelength source and, for each incident light wave of predetermined wavefront, the interference signals of said second plurality ($N_\omega$) of interference signals are acquired for $N_\omega$ central wavelengths of said incident light wave and a fixed path difference between the object arm and the reference arm of the first interferometer.

9. The optical characterization method as claimed in any one of claims 1 to 7, wherein:

- for each incident light wave of given wavefront, the interference signals of said second plurality ($N_\omega$) of interference signals are acquired for $N_\omega$ path differences between the object arm and the reference arm of the first interferometer.

10. The optical characterization method as claimed in any one of the preceding claims, wherein the $N_{in}$ incident light waves are spatially coherent and have wavefronts controlled by means for spatially shaping the wavefront.

11. The optical characterization method as claimed in claim 10, wherein said $N_{in}$ incident light waves of the first plurality of light waves are plane waves each having a wave vector with a different direction.

12. The optical characterization method as claimed in any one of claims 1 to 9, wherein said light source is a low spatial coherence source, the method further comprising:

- generating, on the basis of each spatially incoherent or partially coherent light wave from the light source, by means of a second interferometer, two polarized illumination waves with orthogonal polarizations and having a spatial shift in a plane conjugate with a focal plane (FP) of the first microscope objective;
- varying said spatial shift to generate the $N_{in}$ incident waves of different wavefronts;
- sending, for each spatial shift, said polarized waves with orthogonal polarizations to the object and reference arms of said first interferometer, respectively, by means of a polarization splitter element (1121);
- acquiring, by means of the detector (1140), said second plurality ($N_\omega$) of interference signals, each interference signal resulting from the interference, in the detection plane of the detector, between a wave backscattered by the sample illuminated by one of said polarized waves with orthogonal polarizations, and a reference wave generated by the reflection of the other of said polarized waves with orthogonal polarizations by a reference mirror of the reference arm, said interference signals being acquired for $N_\omega$ different path differences between the backscattered wave and the reference wave;
- said polychromatic reflection matrix being determined on the basis of the set of the interference signals ($N_{in} \times N_\omega$) acquired for the $N_{in}$ spatial shifts and $N_\omega$ path differences.

13. A system for the optical characterization of a sample (10) formed of a bulk scattering medium, the system comprising:

- a first interferometer (120) with an object arm and a reference arm, the object arm comprising a first microscope objective (110) with a given field of view in which, in operation, the sample (10) is positioned;
- an illuminating device (130) comprising a wide spectral band light source (132), configured to generate a first plurality ($N_{in}$) of incident light waves having different wavefronts;
- a detector (140) comprising $N_{out}$ elementary detectors, configured to acquire, for each incident light wave of given wavefront, a second plurality ($N_\omega$) of interference signals, each interference signal resulting from the interference, in a detection plane of the detector, between a wave backscattered by the sample illuminated by said incident light wave and a reference wave from the reference arm, said interference signals being acquired for $N_\omega$ different frequencies or $N_\omega$ different path differences between the backscattered wave and the reference wave;

- a processing unit (150) configured to:

- determine a three-dimensional polychromatic reflection matrix $\tilde{\mathbf{R}}_{\rho u} = [\tilde{R}(\rho_{\mathbf{out}}, \mathbf{u_{in}}. \omega)]$ of size $N_{in} \times N_{out} \times N_\omega$ said three-dimensional polychromatic reflection matrix comprising all of the interference signals ($N_{in} \times N_\omega$) acquired for the $N_{in}$ incident light waves and $N_\omega$ frequencies;

- numerically determine, by applying a first propagator, on the basis of said polychromatic reflection matrix, at least a first focused volumetric reflection matrix ($\mathbf{R_{rr}}$), comprising a set of responses for the sample between source points and receiving points that are conjugate with voxels $\mathbf{r_{in}}(x'_{in}, y'_{in}, z_{in})$ and $\mathbf{r_{out}}(x'_{out}, y'_{out}, z_{out})$ of the sample (10) which are located at depths $z_{in}$ and $z_{out}$ in the sample, respectively;

- determine, on the basis of said first focused volumetric reflection matrix, at least one map of a physical parameter of said sample.

14. The optical characterization system as claimed in claim 13, wherein said first interferometer is a Linnik interferometer and the reference arm comprises a reference mirror and a second microscope objective.

15. The system as claimed in any one of claims 13 and 14, wherein:

- the light source is a low spatial coherence source; and the illuminating device (1130) comprises
- a second interferometer configured to generate, on the basis of each spatially incoherent or partially coherent light wave from the light source, two polarized illumination waves with orthogonal polarizations and having a spatial shift in a plane conjugate with a focal plane (FP) of the first microscope objective (110); and
- means for varying the spatial shift; and wherein
- said first interferometer (1120) is a Linnik interferometer and comprises
- a polarization splitter element (1121) configured to send, to the object and reference arms, respectively, each of said polarized waves with orthogonal polarizations and having said spatial shift;
- means for varying the path difference between the reference arms; and wherein
- each interference signal of said second plurality ($N_\omega$) of interference signals results from the interference, in the detection plane of the detector, between a wave backscattered by the sample illuminated by one of said polarized waves with orthogonal polarizations, and a reference wave generated by the reflection of the other of said polarized waves with orthogonal polarizations by a reference mirror of the reference arm, said interference signals being acquired for $N_\omega$ different path differences between the backscattered wave and the reference wave;
- said polychromatic reflection matrix is determined on the basis of the set of the interference signals ($N_{in} \times N_\omega$) acquired for the $N_{in}$ spatial shifts and $N_\omega$ path differences.

FIG.1A

FIG.1B

$\mathbf{u_{in}} = (v_{in}, w_{in})$

$\Delta_u$

$\delta u_{in}$

201

n

m

**FIG.2A**

$\rho_{out} = (x_{out}, y_{out})$

202

$\delta p_{out}$

$\Delta_\rho$

p

q

**FIG.2B**

$i = p+(\sqrt{N_{out}}-1) \times q$

$R(\mathbf{u_{in}}, r_{out}, \omega)$

$j = n+(\sqrt{N_{in}}-1) \times m$

$N_\omega$

k

$\omega$

1

$\mathbf{u_{in}}$

**FIG.2C**

$r_{out}$

$$\mathbf{r_{in}} = (\rho'_{in}, z_{in}) \qquad \mathbf{r_{out}} = (\rho'_{out}, z_{out})$$
$$\rho'_{in} = (x'_{in}, y'_{in}) \qquad \rho'_{out} = (x'_{out}, y'_{out})$$

FIG.3A

$R(\rho'_{out}, z_{out}, \rho'_{in}, z_{in})$   301

$\rho_{in}$

$\rho'_{out}$

$y'_{out} - y'_{in}$

$x'_{out} - x'_{in}$   302

Image confocale   303

$r_{in} = r_{out}$

$y'_{out}$

$x'_{out}$

RPSF

$y'_{out} - y'_{in}$

$x'_{out} - x'_{in}$

305

Image I'(r)   304

FIG.3B

EP 4 558 785 B1

FIG.3C

FIG.4A

FIG.4B

FIG.5

FIG.6

Image confocale
En-face

z = 250 μm

# FIG.7A

$\rho_{ms}$ = taux de diffusion multiple

# FIG.7B

# FIG.7C

71

Section XZ (y'=0 μm)

FIG.7D

FIG.8

FIG.9

FIG.10A

FIG.10B

EP 4 558 785 B1

FIG.11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20040061867 A

- US 20210310787 A

**Littérature non-brevet citée dans la description**

- **S. M. POPOFF et al.** Measuring the Transmission Matrix in Optics. *Phys. Rev. Lett.*, 2010, vol. 104, 100601

- **A. BADON et al.** Smart optical coherence tomography for ultra-deep imaging through highly scattering media. *Sci. Adv.*, 2016, vol. 2, e1600370